# EUROPEAN PATENT SPECIFICATION

(11) **EP 0 380 018 B1**
(45) Date of publication and mention of the grant of the patent: **19.07.1995**
(21) Application number: 90101207.0
(22) Date of filing: 22.01.1990
(51) Int. Cl.: C12P 21/08, C12N 5/20, C12N 15/06, A61K 39/395, G01N 33/577

(54) **Rat monoclonal antibodies directed against human antigens and processes for preparation thereof**
Monoklonale Antikörper von Ratten gegen menschliche Antigene und Verfahren zu deren Herstellung
Anticorps monoclonaux de rat contre des antigènes humains et méthode pour les préparer

(30) Priority: 23.01.1989 US 299694
(43) Date of publication of application: 01.08.1990
(73) Proprietor: ABBOTT LABORATORIES, Abbott Park, Illinois 60064-3500 (US)
(72) Inventor: Bazin, Herve, B-1200 Brussels (BE); De Bruyere, Marc, B-1170 Brussels (BE)
(74) Representative: Modiano, Guido, Dr.-Ing.

(56) References cited:
- PROCEEDINGS OF THE NATL. ACADEMY OF SCIENCES USA, vol. 76, no. 8, August 1979; E.L.REINHERZ et al., pp. 4061-4065#
- JOURNAL OF IMMUNOLOGY, vol. 132, no. 6, June 1984, American Association of Immunologists, US; A. PIERRES et al., pp. 2775-2782#
- JOURNAL OF IMMUNOLOGY, vol. 131, no. 5, November 1983, American Association of Immunologists, US; D.P. DIALYNAS et al., pp. 2445-2451#

## Description

The present invention relates to the production of rat monoclonal antibodies. More particularly, the present invention relates to a new, reproducible method of producing rat monoclonal antibodies that bind to human T-lymphocyte antigen epitopes other than those epitopes defined by murine monoclonal antibodies directed to the same antigen and rat monoclonal antibodies that bind to the human CD4 antigen. The present invention also relates to the hybridomas produced by the novel methods and to the monoclonal antibodies secreted by the hybridomas. The antibodies are useful for detecting the human antigens in vitro, and in particular CD4 which is useful for determining the prognosis of AIDS infection. The antigens are expected to be useful for the treatment of diseases, such as AIDS, wherein the antigens are involved in the disease process. The antibodies are also expected to be useful in isolating the antigens.

### BACKGROUND OF THE INVENTION

The development of monoclonal antibodies (MAbs) has offered the opportunity of studying the distribution, structure and function of T-lymphocyte membrane antigens. Over the past years a series of molecules present on the T-lymphocyte have been defined by mouse monoclonal antibodies; some have been shown to be associated with T-lymphocyte function. For instance, T-cell antigen receptor complex (Ti-T3) is recognized by CD3 monoclonal antibodies (e.g. OKT3) and by WT311 (Kung, P.C. et al, Science, 206, 347, 1979); the receptor for Interleukin-2 is recognized by CD25 monoclonal antibodies (e.g., TAC) (Leonard, W.J. et al. Nature, 300, 267, 1982); and the T-lymphocyte receptor for sheep erythrocytes (E-receptor), which has recently been proposed as a ligand for LFA-3, is recognized by CD2 monoclonal antibodies (e.g. OKT11) (Verbi, W. et al Eur. J. Immunol., 12, 81, 1982 and Selvaraj, T. et al, Nature, 326, 400, 1987). Some antibodies to T-lymphocytes mimic the effect of natural ligands or of antigen stimulation: OKT3 and WT31 activate T-lymphocytes through the T-cell antigen receptor complex (van Wauwe, J.P. et al, J. Immunol., 124, 2708, 1980 and Meuer, S.C. et al, J. Exp. Med., 158, 988, 1983). Certain combinations of CD2 monoclonal antibodies activate T-lymphocytes through an alternative pathway (Meuer, S. et al. Cell, 36, 897, 1984). CD4 and CD8 monoclonal antibodies (e.g., OKT4 and OKT8) (Reinherz, E.L. et al, Proc. Natl. Acad. Sci. USA, 76, 4061, 1979 and Reinherz, E.L. et al, J. Immunol., 124, 1301, 1980) define distinct subpopulations of T-lymphocytes. The antigen defined by CD4 monoclonal antibodies is mainly expressed on T-helper cells and seems to recognize MHC class II gene products, while the antigen defined by CD8 monoclonal antibodies is mainly expressed to T suppressor/cytotoxic cells and seems to recognize MHC class I antigens (Meuer, S.C. et al, Proc. Natl. Acad. Sci. USA, 79, 4395, 1982 and Biddison, W.E. et al, J. Immunol., 131, 152, 1983).

In addition to their role in regulating immune function, T-cells bearing the CD4 antigen (T-4 cells) are useful in the diagnosis of disease. In particular, the Human Immunodeficiency Virus (HIV) attacks T-4 cells resulting in decline in the number of circulating T-4 cells. A decline in the number of circulating T-4 cells is a predictor of the development of Acquired Immunodeficiency Syndrome (AIDS) (Goedert, J. et al, JAMA, 257, 331-334, 1987). Therefore, the enumeration of T-4 cells is medically useful in the management of patients infected with HIV.

Antibodies against the CD4 antigen are thought to be useful in treating disorders of the immune system. For instance, in patients having rheumatoid arthritis or psoriatic arthritis, the intravenous injection of murine anti-CD4 monoclonal antibodies gave objective relief to the patients (Herzog, C. et al, The Lancet, December 19, 1987, 1461-1462).

Murine monoclonal antibodies which recognize the CD4 antigen characteristic of human T-4 cells has been described by Reinherz, E. et al., Proc. Natl. Acad. Sci. USA, 76(8), 4061-4065, 1979.

Despite the development of these mouse monoclonal antibodies, there has been an interest in producing anti-human T-lymphocyte and especially anti-human CD4 monoclonal antibodies in a rat model because of the difference in immune repertoire between rat and mouse. Moreover, because large quantities of rat monoclonal antibodies are easily produced and purified and because some rat immunoglobulin isotypes have been shown to fix human complement and to activate human killer cells, rat monoclonal antibodies are attractive reagents for clinical therapeutic use.

As background art to the present invention, Pierres, A. et al, The Journal of Immunology, 132(6), 2775-2782, 1984 describes the production of rat monoclonal antibodies.

### SUMMARY OF THE INVENTION

Accordingly, one object of the present invention is to provide a reproducible method for producing novel rat monoclonal antibodies to human T-cell lymphocyte antigen which bind to a CD4 antigen epitope distinct enough from that bound by murine monoclonals so there is no cross blocking.

Another object of the present invention is to provide a hybridoma capable of producing novel rat monoclonal antibodies that bind to human T-cell lymphocyte antigens at a CD4 antigen epitope distinct enough from that bound by murine monoclonals so there is no cross blocking.

A further object of the present invention is to provide a novel rat monoclonal antibody that binds to human T-cell lymphocyte antigens at a CD4 antigen epitope distinct enough from that bound by murine monoclonals so there is no cross blocking.

An even further object of the present invention is to provide a rat monoclonal antibody that binds human T-cell lymphocyte antigens at a human CD4 antigen epitope other than those epitopes defined by the known anti-T4 murine antibodies including anti-CD4 murine monoclonal antibodies OKT4 and Leu3a.

These and other objects of the present invention have been achieved by providing a method of producing rat monoclonal antibodies that bind to human CD4 antigen, the method comprising:
(1) immunizing rat or rat immunocompetent cells in vitro with an immunogenic amount of an antigen comprising CD4 molecule;
(2) fusing immunized cells from said rat or immunized rat immunocompetent cells with immunocytoma cells;
(3) selecting hybridoma cells that produce antibody that binds to a human CD4 antigen epitope distinct enough from that bound by murine monoclonals so there is no cross blocking;
(4) culturing the selected hybridoma cells; and
(5) recovering said antibody.

In another embodiment, the present invention provides a hybridoma that produces a rat monoclonal antibody that binds to a human CD4 antigen epitope distinct enough from that bound by murine monoclonals so there is no cross blocking.

In still another embodiment, the present invention provides a rat monoclonal antibody that binds to a human CD4 antigen epitope distinct enough from that bound by murine monoclonals so there is no cross blocking.

In a preferred embodiment, the rat monoclonal antibody binds to a human CD4 antigen epitope other than those epitopes defined by murine monoclonal antibodies OKT4 and Leu3a.

In an even further embodiment, the present invention provides a binding protein having the binding specificity of anti-CD4 rat monoclonal antibody LO-CD4-a or anti-CD4 rat monoclonal antibody LO-CD4-b produced by hybridomas LO-CD4-a and LO-CD4-b, respectively, having ECACC Deposit Nos. 89012101 and 89012102, respectively.

The present invention additionally provides the use of a rat monoclonal antibody produced according to the above-described methods for manufacturing a preparation for therapeutic use.

The present invention also provides an in vitro diagnostic method comprising contacting a rat monoclonal antibody produced according to the above-described methods with a suitable test sample and assaying for the antigen.

### BRIEF DESCRIPTION OF THE FIGURES

Figure 1A shows the reactivity pattern of monoclonal antibodies LO-CD4-a and LO-CD4-b and of reference pan-T or subset T specific monoclonal antibody OKT3, T11, T12, LO-CD5-a and OKT4 with peripheral blood lymphocytes (PBL) (left panel) and phytohemagglutinin (PHA) activated PBL (right panel). X-axis: fluorescence intensity; Y-axis: cell number.

Figure 1B shows the reactivity pattern of monoclonal antibodies LO-CD4-a and LO-CD4-b and of reference pan-T or subset T specific monoclonal antibody OKT3, T11, T12, LO-CD5-a and OKT4 with tonsil cells (left panel) and thymocytes (right panel). X-axis: fluorescence intensity. Y-axis: cell number.

Figure 2 shows patterns from double labeling with pan-T or pan-B monoclonal antibodies and rat monoclonal antibodies. Tonsil cells are labeled with monoclonal antibodies LO-CD4-a plus fluorescein-conjugated rabbit anti-rat immunoglobulin antibody (Fab′)₂ fragments (RARA-FITC) followed by mouse anti-CD2-phycoerythrin conjugate (T11PE) or mouse anti-CD19 phycoerythrin conjugate (B4PE).

Figure 3 is double labeling patterns that show subsets of lymphocytes recognized by LO-CD4-a and LO-CD4-b. Peripheral blood mononuclear cells (PBMN) were labeled with rat monoclonal antibodies LO-CD4-a or LO-CD4-b plus RARA-FITC followed by OKT4PE or OKT8PE.

Figure 4 shows flow cytometer scans of fluorescent labeled PBLs. The X-axis in all panels represents green fluorescence (Fluorescence 1) or red fluorescence (Fluorescence 2) intensity and the Y-axis in all panels represents counts full scale. Figs. 4(A) to 4(G) show the data for monoclonal antibody LO-CD4-a and Figs. 4(H) to 4(N) show the data for monoclonal antibody LO-CD4-b.

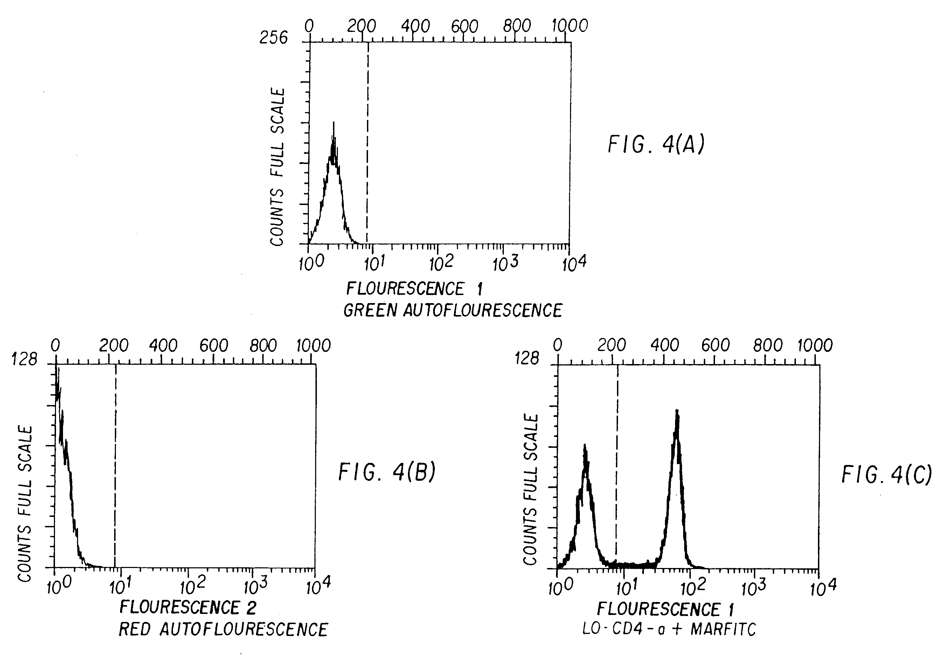

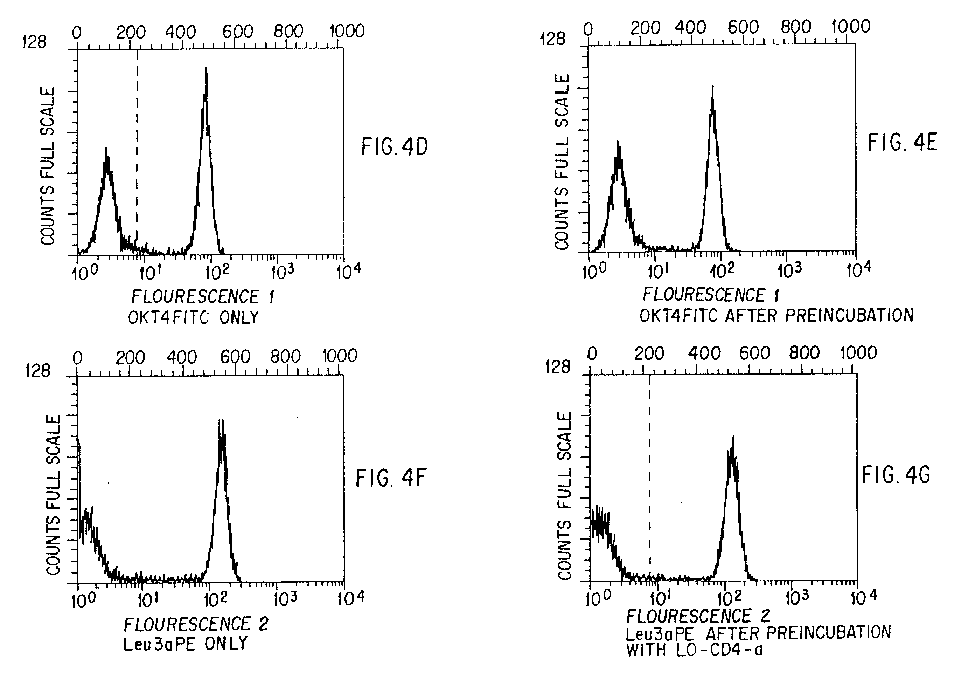

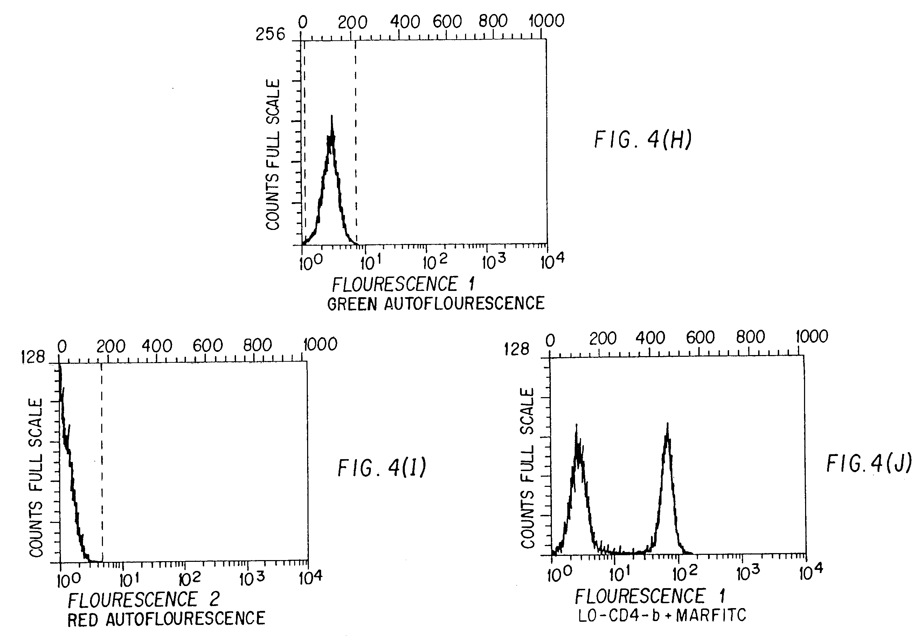

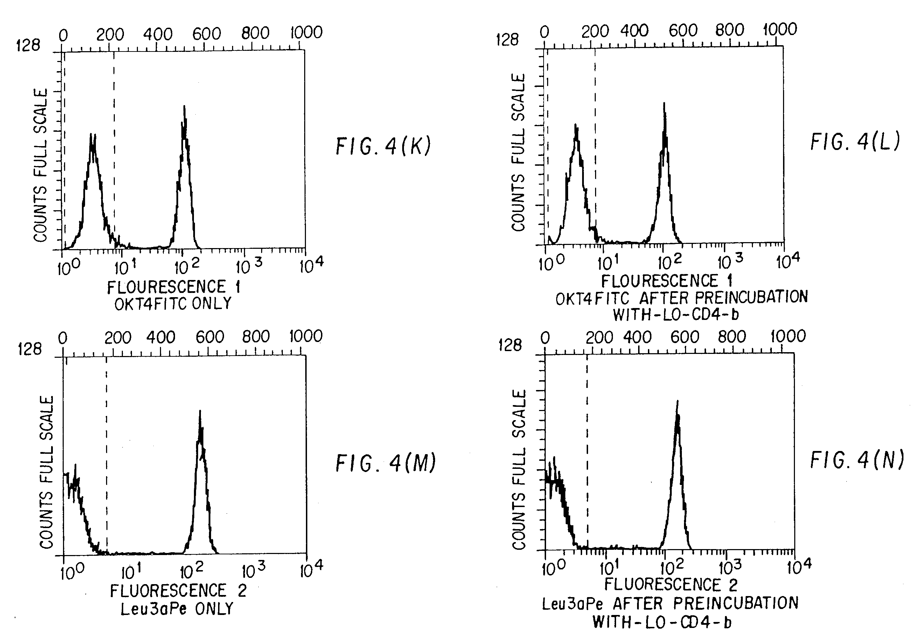

Figs. 4(A) and 4(B) are controls showing green and red autofluorescence, respectively, for PBLs in the absence of added antibody conjugate. Fig. 4(C) is a control showing the binding of monoclonal antibody LO-CD4-a to PBLs detected by fluorescein conjugated mouse anti-rat conjugate (MARFITC). Fig. 4(D) is a control showing the fluorescence intensity of PBLs after incubation with OKT4FITC only. Fig. 4(E) shows the fluorescence intensity of PBLs incubated with OKT4FITC after preincubation with LO-CD4-a. Fig. 4(F) is a control showing the fluorescence intensity of PBLs after incubation with Leu3apE only. Fig. 4(G) shows the fluorescence intensity of PBLs incubated with Leu3apE after preincubation with LO-CD4-a.

Figs. 4(H) to 4(N) parallel Figs. 4(A) to 4(G), except that where monoclonal antibody LO-CD4-a was used in Figures 4(A) to 4(G), monoclonal antibody LO-CD4-b was used in Figures 4(H) to 4(N).

Figure 5 shows flow cytometer scans of fluorescent labeled PBLs. The X-axis in all panels represents green fluorescence (Fluorescence 1) or red fluorescence (Fluorescence 2) intensity and the Y-axis in all panels represents counts full scale. Figs. 5(A) to 5(H) show the data for undepleted PBLs and Figs. 5(I) to 5(P) show the data for CD4 depleted PBLs.

Figs. 5(A) to 5(B) are controls showing green and red autofluorescence, respectively, for PBLs in the absence of added antibody conjugate. Figs. 5(C) and 5(D) show binding of monoclonal antibodies LO-CD4-a and LO-CD4-b, respectively, to undepleted PBLs detected by phycoerythrin conjugated goat anti-rat antibody (GARPE). Fig. 5(E) is a control showing immunofluorescence of PBLs after incubation with GARPE.

Figs. 5(F), 5(G) and 5(H) show the fluorescence intensity of undepleted PBLs after incubation with Leu3aPE, OKT4FITC and OKT8FITC, respectively.

Figs. 5(I) to 5(P) parallel Figs. 5(A) to 5(H), except that depleted PBLs were used in place of undepleted PBLs.

Figure 6 shows dot plot analyses of flow cytometry data of red fluorescence vs. green fluorescence for fluorescent labeled PBLs. The X-axis of each plot represents green fluorescence (Fluorescence 1), and the Y-axis of each plot represents red fluorescence (Fluorescence 2).

Figs. 6(A) to 6(D) show the data for monoclonal antibody LO-CD4-b, and Figs. 6(E) to 6(I) show the data for monoclonal antibody LO-CD4-a.

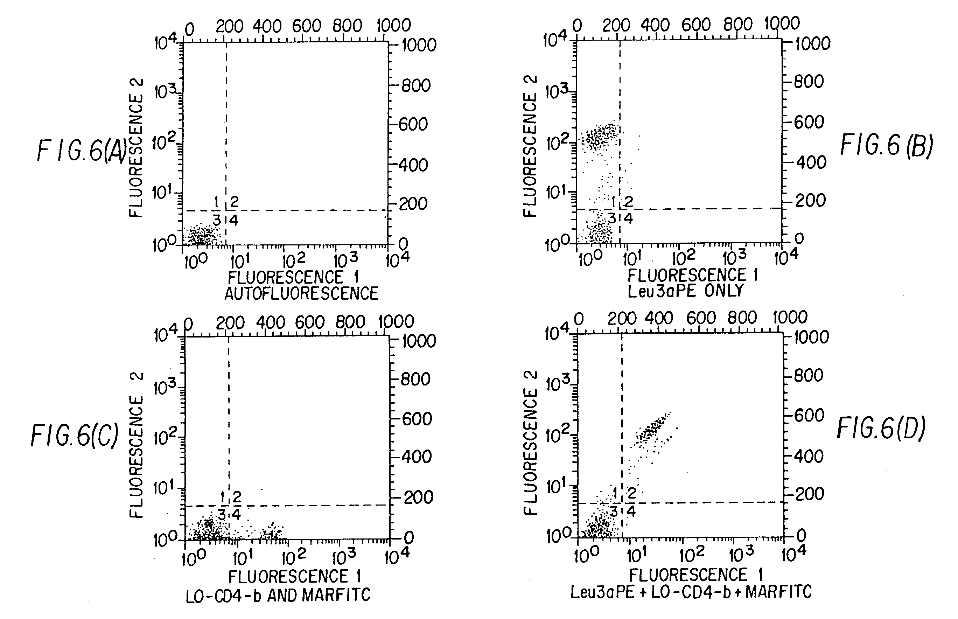

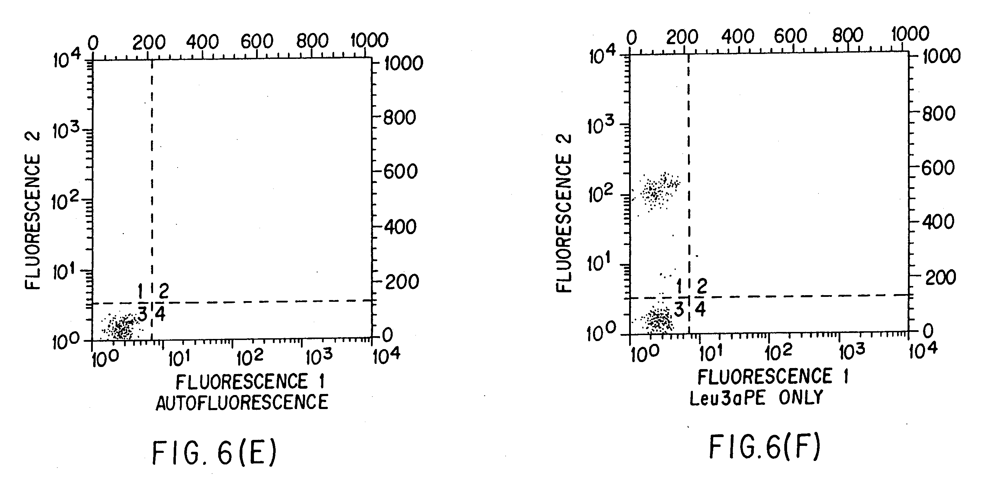

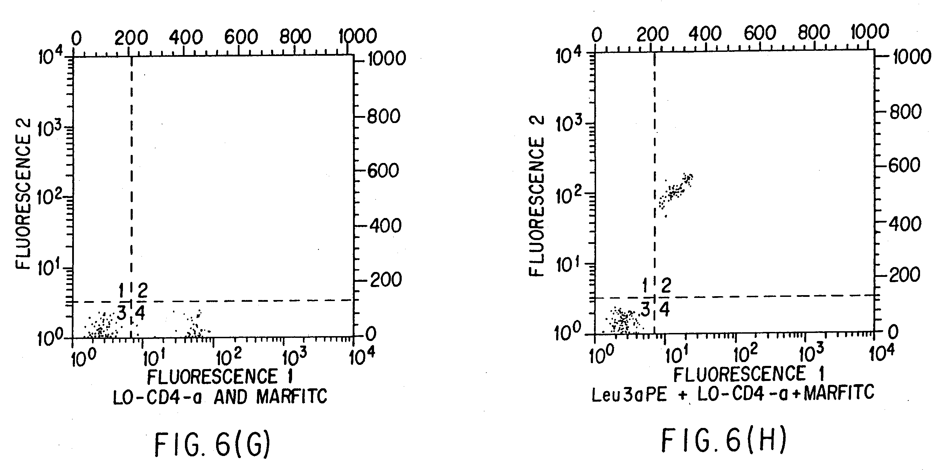

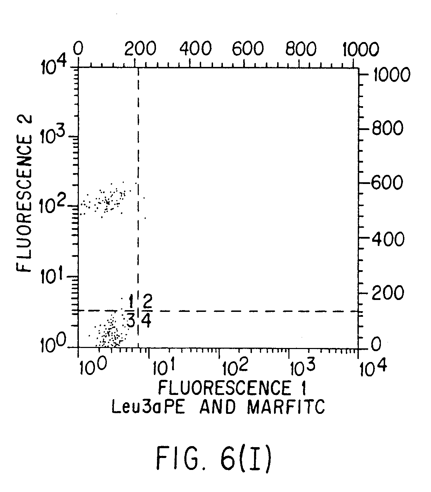

Fig. 6(A) is a control showing the dot plot for red and green autofluorescence of PBLs in the absence of added antibody conjugate. Fig. 6(B) shows the dot plot for fluorescence of PBLs incubated with monoclonal antibody conjugate Leu3aPE. Fig. 6(C) shows the dot plot for fluorescence of PBLs incubated with MARFITC after preincubation with monoclonal antibody LO-CD4-b. Fig. 6(D) shows the dot plot for fluorescence of PBLs incubated with monoclonal antibody conjugates Leu3aPE and then MARFITC after preincubation with LO-CD4-b.

Figs. 6(E) to 6(H) parallel Figs. 6(A) to 6(D), except that monoclonal antibody LO-CD4-a was used instead of monoclonal antibody LO-CD4-b. Fig. 6(I) shows the dot plot for fluorescence of PBLs incubated with MARFITC after preincubation with Leu3a.

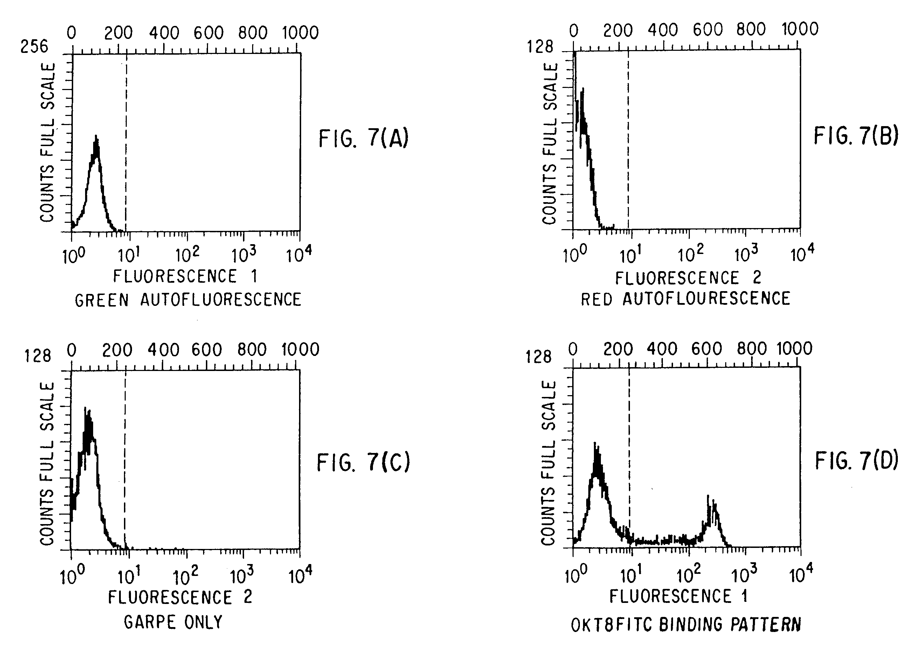

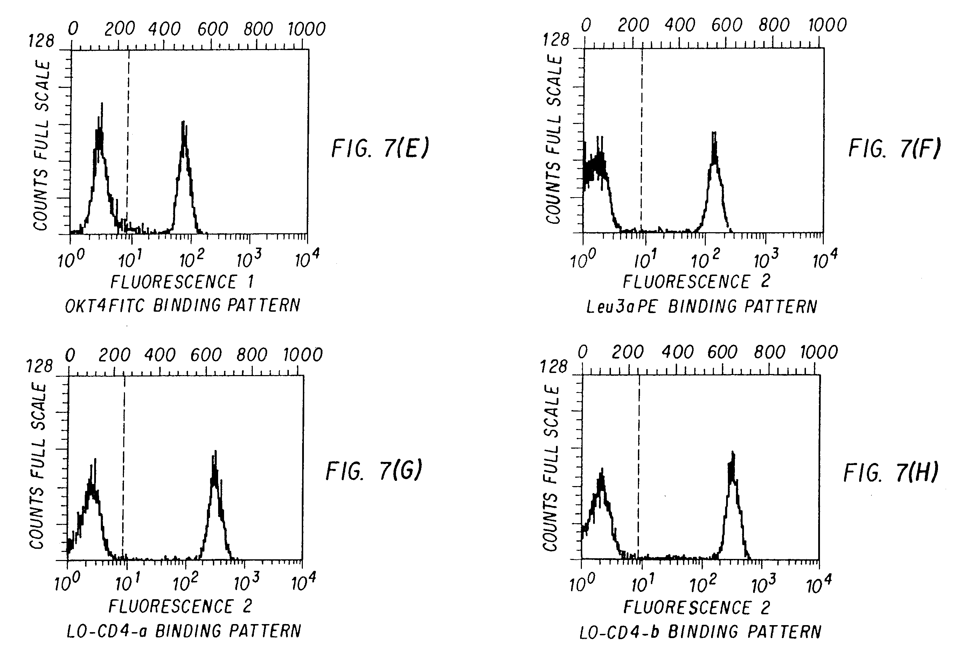

Figure 7 shows flow cytometer scans of fluorescent labeled PBLs. The X-axis in all panels represents green fluorescence (Fluorescence 1) or red fluorescence (Fluorescence 2) intensity and the Y-axis in all panels represents counts full scale.

Figs. 7(A) and 7(B) are controls showing green and red autofluorescence, respectively, for PBLs in the absence of added antibody conjugate. Fig. 4C is a control showing the fluorescence for PBLs incubated only with GARPE. Fig. 7(D) shows the fluorescence intensity of PBLs incubated with OKT8 as detected by phycoerythrin conjugated goat anti-mouse antibody (GAMPE). Figs. 7(E) and 7(F) show the fluorescence intensity of PBLs incubated with OKT4 and Leu3a, respectively, as detected by GAMPE. Figs. 7(G) and 7(H) show the fluorescence intensity of PBLs incubated with LO-CD4-a and LO-CD4-b, respectively, as detected by GARPE.

### DETAILED DESCRIPTION OF THE INVENTION

For the purposes of this application, the following abbreviations have the following meanings: AET: 2-aminoethylisothiuronium; ALL: acute lymphoblastic leukemia; CALL: common ALL; CLL: chronic lymphocytic leukemia; conA: concanavalin A; Ig: immunoglobulin: DMSO: dimethylsulfoxide; E: sheep erythrocyte rosette; GAM-FITC: fluorescein-conjugated goat anti-mouse Ig antibodies; IL2: interleukin-2; PE: phycoerythrin; FITC: fluorescein isothiocyanate; kDa: kilodalton; MAb: monoclonal antibody; MLR: mixed lymphocyte reaction; NBCS: newborn calf serum; NHL: non Hodgkin lymphoma; PBS: 0.15 NaCl buffered at pH 7.2 with 8 mM phosphate; PHA: phytohemagglutinin; PWM: pokeweed mitogen; PAGE: polyacrylamide gel electrophoresis; PBMN: peripheral blood mononuclear cells; PMSF: phenyl methane sulfonyl fluoride; RARA-FITC: fluorescein-conjugated rabbit anti-rat IG antibody F(ab')₂ fragments; SAC: staphylococcus aureus Cowan I; SDS: sodium dodecyl sulfate; SRBC: sheep red blood cells; TT: tetanus toxoid; ECACC: European Collection of Animal Cell Cultures, PHLS Centre for Applied Microbiology and Research at Porton Down, Salisbury, Wilts. SP 4-OJG, United Kingdom.

As described above, the present invention provides a method of producing rat monoclonal antibodies that bind to human CD4 antigen, the method comprising:
(1) immunizing a rat or rat immunocompetent cells in vitro with an immunogenic amount of an antigen comprising CD4 molecule;
(2) fusing immunized cells from said rat or immunized rat immunocompetent cells with immunocytoma cells;
(3) selecting hybridoma cells that produce antibody that binds to a human CD4 antigen epitope distinct enough from that bound by murine monoclonals so there is no cross blocking;
(4) culturing the selected hybridoma cells; and
(5) recovering said antibody.

A variety of rats that are easily available to researchers in the art, such as Wistar, Sprague-Dawley, Lewis and Louvain rats, are suitable for use as the source of immunized cells for fusion.

LOU/C or LOU/M rats are a preferred source of immunized cells for fusion. These rats are syngeneic rats. These two strains of rats were originally developed by Bazin et al (J. Natl. Cancer Inst._(U.S.), 51, 1359, 1973). The strains are readily available to those working in the art.

Methods for immunizing the rats are described below in detail.

Alternatively, stimulation of antigen-specific B-cells can be accomplished in vitro. Rat immunocompetent cells are harvested from lymphatic organs removed from the rat. Procedures for harvesting and in vitro immunization are well known to artisans in the field. (C.L. Reading, Methods in Enzymology, 121:18-27, 1986; D. Gratecos et al., J. Immunological Methods 103, 169-178, 1985; B.B. Mishell, S.M. Shiigi, Selected Methods in Celluar Immunology, 1980).

The immunogen can be derived from any T-lymphocyte or any T-cell line. Human origin of the cells is preferred. Most preferably, CD4 would be obtained from circulating human T lymphocytes. Purified antigen can also be used.

In a preferred embodiment, the method is directed to producing rat monoclonal antibodies to human CD4 antigen.

CD4 was originally defined by the OKT-4 antibody (Kung, P., et al, Science, 206, 347, 1979) as an antigen on the surface of a subpopulation of T cells present in circulating peripheral blood lymphocytes. The CD4 antigen has been further characterized as a glycoprotein of 55kD with structural relationships to other members of the immunoglobulin family (Thompson, J., and Zimmermann, W., Tumor Biol., 9, 63-83, 1988). The gene for CD4 has been cloned and the antigen has been expressed on cells other than human T-cells (Maddon, P. et al, Cell, 47, 333-348, 1986). The CD4 antigen has also been expressed in soluble form, making it routine to isolate the glycoprotein in pure form (Deen, K. et al, Nature, 331, 82-84, 1988.) The CD4 antigen is also expressed on T-cell lines such as CEM and HPB-ALL as well as other non-T-cell lines such as U937 of myeloid origin.

Reference to CD4 antigen refers to the 55kD glycoprotein, or fragments thereof regardless of its form or mode of expression. This includes CD4 on circulating T-cells as well as on circulating non-T-cells such as myeloid cells. Also included are CD4 from other species which would result in rat antibodies which cross-react with human CD4. Further included is recombinant CD4 whether expressed in a human or non-human cell. Additionally, included is purified CD4, whether from natural or recombinant sources.

One preferred example of a suitable T-cell line useful as an immunogen is the T-ALL line and derivatives thereof which are publicly available. Especially preferred is HPB-ALL. (Morikawa, S. et al, Int. J. Cancer, 21, 166, 1978).

The T-cell lines are cultured by known methods appropriate to the particular T-cell line being used.

HPB-ALL can be cultured at 37°C in the presence of 5% CO₂ in RPMI-1640 medium containing 10% fetal calf serum, 2 mM glutamine and streptomycin (PS).

Purified T lymphocytes can be obtained as follows.

Peripheral blood mononuclear cells (PBMN) from normal donors are isolated by centrifugation on Ficoll®-Hypaque (density: 1.077). T lymphocytes are then purified by rosetting according to known methods with 2-aminoethylisothiouronium-treated sheep red blood cells (SRBC-AET). PBMN at a concentration of 5 x 10⁶/ml are then mixed with 1/10 volume SRBC-AET at a concentration of about 1.0 x 10⁹/ml and incubated at room temperature for 15 minutes. The incubation mixture is centrifuged at 100xg for 5 minutes and after resuspending the pellet in a physiological buffer the suspension is incubated at 4°C for 1 to 2 hours. Rosetted cells (greater than 95% of T lymphocytes) are then separated from non-rosetted cells (less than 5% T lymphocytes) by centrifugation on Ficoll®-Hypaque. T-cells are recovered from the rosette by lysing the SRBC with 0.83% NH₄Cl, and then washing with physiological buffer.

Immunization protocols for rats are antigen dependent and can be readily determined by the skilled artisan. In general, a suitable immunization procedure consists of two intraperitoneal injections, the second after a three to four week interval, and, optionally an intraperitoneal booster injection six to eight weeks later.

Concentrations of antigens suitable for immunizing the rats are well known to artisans in the field. (J.J. Langone and H.V. Vunakis, Methods in Enzymology, 1986; Frontiers in Biology, 25, F. Borex, 1981).

The immunogen may be administered along with a pharmaceutically acceptable carrier such as Freunds adjuvant or saline.

As used herein "immunized cells" refers to the sensitized spleen cells of the immunized rat. The sensitized spleen cells are isolated from the rat by conventional methods. In addition "immunized cells" refers to in vitro immunized immunocompetent cells. The immunized immunocompetent cells are harvested from immune organs by conventional methods.

To produce the hybridoma, the immunized cells or immunized rat immunocompetent cells are fused with immunocytoma cells.

For fusion with immunized cells from LOU rats, IR983F non-secreting rat immunocytoma cells are preferably used. The IR983F fusion cell line (sometimes referred to as the "983 cell line") is a permanent azaquanine-resistant in vitro cell line (Bazin, H., in Protides of the Biological Fluids, H. Pecters. ed., 615, Pergamon, Oxford, 1982) and is publicly available. IR983F cells can also be used as a fusion partner for immunized rat immunocompetent cells.

Other fusion partners for immunized rat cells or immunized rat immunocompetent cells are Y3-AG 1.2.3, YB2/3HL.B2.G11.16AG.20, and YB2/3.0.AG.20 cell lines, all of which are also publicly available. Fusion partners for rat immunocompetent cells may be from another species, such as mouse.

The cells are grown at 37°C in the presence of 5% CO₂. The skilled artisan can readily determine suitable culture conditions for other cell lines (J.J. Langone and H.V. Vanakis, Methods In Enzymology, supra).

Fusion is performed about four days after the last injection of immunogen. Fusion is performed according to many known methods, such as PEG, electrofusion and immunochemical and biochemical methods (J. Immunological Methods, 101, 153-170, 1987, S.R. Samoilovich et al.).

When hybrid clones are observed generally about the seventh day after fusion, they are transferred to 24-well plates. When supernatants in the wells become yellow, screening for specific antibody-secreting hybrid clones can be performed by any suitable method such as indirect immunofluorescence using the immunizing cells as target cells. After a second screening of the whole blood, those hybrid clones which secrete antibodies reacting with lymphocytes, and not with granulocytes and/or monocytes, are selected. The hybrid lines are then cloned twice by limiting dilution. The hybridoma cells are either cultured in culture medium, i.e., complete DMEM, frozen in complete DMEM-7.5% DMSO and stored in liquid nitrogen by known methods (Soding, J.W., Monoclonal Antibodies: Principles and Practice, 86, Academic Press, London, 1984), or injected subcutaneously into LOU rats or into LOU/C·IgK-1b(OKA) rats to induce solid tumors.

LOU/C·IGK-1b(OKA) rats are a congenic line to the LOU/C strain, having the kappa light chain locus from the OKA strain in LOU/C background (Bazin, H. et al, J. Immunol. Methods, 71, 9, 1984). The tumor cells can be frozen in PBS- 10% FCS-7.5% DMSO and stored in liquid nitrogen by known methods (Bazin, H. et al Int. J. Cancer, 10, 568, 1972). The tumor cells can also be injected intraperatoneally to induce ascitic tumor. The cells can be collected from the ascitic fluid and stored at -80°C.

The rat monoclonal antibodies can be produced in quantity by known methods, either in vitro (See, for example, Bodeus, M. et al, Immunol. Meth., 79, 1, 1985) or in vivo (See, for example, Bazin, H. et al, Int. J. Cancer, 10, 568, 1972 and Bazin, H., Adv. Cancer Res., 50, 279, 1987).

Purification of the rat monoclonal antibodies can also be achieved by known methods (See, for example, Bazin, H., J. Immunol. Meth., 71, 9, 1984).

According to the above described methods, hybridomas that produce rat monoclonal antibodies that bind to human CD4 antigen can be produced. Further it is possible to produce hybridomas that produce monoclonal antibodies that bind to T-cell lymphocyte antigen epitopes other than those epitopes defined by murine monoclonal antibodies directed to the same antigen or antibodies that bind to a human CD4 antigen epitope other than those epitopes defined by the well known murine monoclonal antibodies OKT4 and Leu3a.

These latter monoclonal antibodies can be identified by performing conventional inhibition assays. A description of one suitable assay is described in detail in Example 5 below.

Two preferred rat monoclonal antibodies having the identifying characteristics that: (1) they bind to human CD4 antigen, and (2) they do not bind to the same CD4 antigen epitopes defined by murine monoclonal antibodies OKT4 and Leu3a, have been isolated according to the above method.

One of these preferred monoclonal antibodies, designated LO-CD4-a, was obtained after immunization with the T-ALL line HPB-ALL. This monoclonal antibody is produced by hybridoma LO-CD4-a which has been deposited at the EPACC and has Deposit No. 89012101.

The other of the preferred monoclonal antibodies has been designated LO-CD4-b and was obtained after immunization with purified T lymphocytes. This monoclonal antibody is produced by hybridoma LO-CD4-b which has been deposited at the EPACC and has Deposit No. 89012102.

A detailed description of the production of the hybridomas producing these antibodies and of further identifying characteristics of these antibodies is set forth in the working Examples below.

A brief summary of the further identifying characteristics of the monoclonal antibodies LO-CD4-a and LO-CD4-b follows.

### Reactivity Pattern of Monoclonal Antibodies LO-CD4-a and LO-CD4-b with Normal Cells

(1) React with:
   (a) about 50% or less of peripheral blood lymphocytes
   (b) about 70% of PHA-activated lymphocytes
   (c) about 60% of tonsil cells
   (d) about 60% of thymocytes
(2) Essentially do not react with:
   (a) monocytes
   (b) granulocytes
   (c) red blood cells
   (d) platelets
   (e) B-lymphocyte-enriched suspension of PBL depleted of T lymphocytes
   (f) B-lymphocyte-enriched suspension of tonsil cells depleted of T lymphocytes.

### Specificity of Monoclonal Antibodies LO-CD4-a and LO-CD4-b as Determined by Double Labeling Experiments

(1) T-lymphocyte specific
(2) Recognize same subpopulation of T lymphocytes recognized by murine monoclonal antibody OKT4 and Leu3a
(3) Recognize different subpopulation of T lymphocytes than is recognized by murine monoclonal antibody OKT8

### Reactivity Patterns of Monoclonal Antibodies LO-CD4-a and LO-CD4-b with Leukemia Cells

(1) Cross react with non-T cell line U937 (expresses the T4 antigen) (Table 3)
(2) Essentially do not react with non-T and non-B lymphoblasts (Table 2)
(3) React with T-ALL cell lines (Table 2)
(4) React with CEM and HPB-ALL T-cell lines (Table 4)
(5) React weakly with MOLT-4 T-cell line (Table 4)
(6) Do not react with JURKAT T-cell line (Table 4)

### Antigenic Specificity of Monoclonal Antibodies LO-CD4-a and LO-CD4-b

Specific only for CD4 positive peripheral blood lymphocytes

### Effects of Monoclonal Antibodies LO-CD4-a and LO-CD4-b on Lymphocyte Proliferation

(1) LO-CD4-a
   (a) Proliferation of lymphocytes induced by tetanus toxoid or allogenic-antigen induced: Inhibited about 40-60%
   (b) Proliferation of lymphocytes induced by conconavalin A, pokeweed mitogen or T3-induced proliferation: Inhibition not significant
   (c) Proliferation of lymphocytes induced by phytohemagglutinin: No inhibition
(2) LO-CD4-b
   (a) Proliferation of lymphocytes induced by tetanus toxoid or allogenic-antigen induced: Inhibited about 70%
   (b) Proliferation of lymphocytes induced by conconavalin A, pokeweed mitogen or T3-induced proliferation: Inhibition weak, i.e., about 30 to 40%
   (c) Proliferation of lymphocytes induced by phytohemagglutinin: No inhibition

Using the first method of the present invention, one may anticipate that other rat monoclonal antibodies can be developed that recognize epitopes on human T-cell lymphocyte antigens distinct from those epitopes recognized by murine monoclonal antibodies directed to the same antigens. For example, thus far investors have only been able to identify rat monoclonal antibodies to human CD8 marker that recognize the same epitope as that recognized by murine monoclonal antibodies such as OKT8. The present method should lead to development of monoclonal antibodies that recognize a broader range of epitopes than currently used methods, when practiced by one skilled in the art.

The present invention also includes a binding protein having the identifying characteristics of monoclonal antibodies LO-CD4-a produced by hybridoma LO-CD4-a having ECACC Deposit No. 89012101, and a binding protein having the identifiying characteristics of monoclonal antibodies LO-CD4-b produced by hybridoma LO-CD4-b having ECACC Deposit No. 89012102.

Recombinant DNA techniques make it possible to construct antibodies having the binding properties of one antibody in the framework of an antibody of a different class or even from a different species. Such techniques are well known in the field (Morrison, S., et al Proc. Natl. Acad. Sci. USA, 81, 6851-6855, 1984 and Jones, P. et al. Nature, 321, 522-525, 1986.) Also included are the so-called "single chain" antibodies in which the binding specificity of a parent antibody is reproduced in a binding protein of about 25 kDa (Bird, R. et al, Science, 242, 423-426, 1988.) All binding proteins having the binding characteristics of LO-CD4-a and LO-CD4-b, whether of natural or recombinant sources, are included in the present invention.

The present invention also provides the use of a rat monoclonal antibody according to the present invention for manufacturing a preparation for an in vivo therapeutic treatment.

The rat monoclonal antibody is generally administered along with a pharmaceutically acceptable carrier. The pharmaceutically acceptable carrier that is used is not critical to the method and the skilled artisan can readily determine suitable carriers.

A pharmaceutically acceptable diluent can also be employed in the therapeutic preparation of the present invention. The particular pharmaceutically acceptable diluent employed is not critical thereto. Examples of such diluents include physiological saline, Ringer's solution, vitamin cocktail and amino acid vitamin cocktail.

The antibodies may be administered intravenously or by any method determined by the skilled artisan to be suitable for the particular therapy.

The pharmaceutically effective amount of the antibodies of the present invention to be administered will vary depending upon the age, weight and sex of the patient and can readily be determined by the skilled artisan.

The present invention further provides an in vitro diagnostic method comprising contacting a rat monoclonal antibody according to the present invention with a suitable test sample and assaying for the antigen.

The term "test sample" means, for example, tissue biopsies, serum, acetic fluid and spinal fluid.

In vitro detection can be carried out using any of the well known in vitro immunological assays, such as those described by Young, W.W. et al, J. Exp. Med., 150, 1008-1019, 1979 and Kannagi, R. et al, Cancer Res., 43, 4997-5005, 1983).

### EXAMPLES

The invention will now be described by reference to the following specific examples which are for illustration only and are not intended to limit the present invention.

### EXAMPLE 1

### PRODUCTION OF HYBRIDOMAS LO-CD4-a AND LO-CD4-b SECRETING ANTI-CD4 MONOCLONAL ANTIBODIES

### Isolation of lymphocytes

Peripheral blood mononuclear cells (PBMN) from normal donors were isolated by centrifugation on Ficoll®-Hypaque (d: 1.077). T lymphocytes were purified by resetting with 2-aminoethylisothiouronium-treated sheep red blood cells (SRBC-AET) as follows: PBMN at 5 x 10⁶/ml were mixed with 1/10 volume AET-SRBC at about 1.0 x 10⁹/ml and incubated at room temperature for 15 minutes. After centrifugation at 100 g for 5 minutes and new incubation at 4°C for 1 or 2 hr, rosetted cells (>95% of T lymphocytes) were separated from non-rosetted cells (<5% T lymphocytes) by centrifugation on Ficoll®-Hypaque. SRBC were lysed with 0.83% NH₄Cl.

### Immunization

Both T cell line HPB-ALL (Morikawa, S. et al. Int. J. Cancer, 21, 166, 1978) and purified T lymphocytes were used for immunization. For the first experiment, 2 x 10⁷ HPB-ALL cells suspended in Freunds Complete adjuvant were injected intraperatoneally (i.p.) in a LOU/C rat. One month later, 2 x 10⁷ cells and seven weeks later, 5 x 10⁷ cells were injected i.p.. An adjuvant was not used for the second two immunogen injections. For the second experiment, 5 x 10⁷ purified T lymphocytes were injected twice i.p. into a LOU/C rat at a three week-interval. For the first injection, the T-lymphocytes were suspended in Freunds Complete adjuvant. No adjuvant was used for the second injection.

HPB-ALL were cultured at 37°C in the presence of 5% Co₂ in RPMI-1640 medium containing 10% fetal calf serum, 2 mM glutamine and PS.

### Production of rat-rat hybridomas

For fusion, IR983F (983) non-secreting rat immunocytoma cells (Bazin, H., "Production of rat monoclonal antibodies with LOU rat non-secreting IR983F myeloma cell line", in Prot. Biol. Fluids, Peeters, H., ed., Pergamon Press, Oxford and N.Y., 1982, 615) were used. The cells were cultured in DMEM medium (Gibco) supplemented with 15% horse serum (HS), 2 mM glutamine, 100 U/ml penicillin, 100 »g/ml streptomycin (PS) and non-essential amino acids (complete DMEM medium). 983 cells were grown at 37°C in the presence of 5% CO₂.

Fusion was performed four days after the last injection.

For the fusion itself, Eagles minimum essential medium or MFM (REGA 3) without L-glutamine (GIBCO), buffered with 10 mM HEPES (GIBCO), and supplemented with 50 mg gentamicin/liter (GIBCO) was used.

The 983 cells grow well in DMEM without HEPES (Dulbecco's modified Eagle's medium; GIBCO) supplemented with 5% heat-inactivated (30 min at 56°C) fetal calf serum (GIBCO), 5% heat-inactivated horse serum (Flow), 1% nonessential amino acids (GIBCO), 1% sodium pyruvate 100 mM: (GIBCO), and 0.1% gentamicin sulfate 50 mg/ml (GIBCO).

All serum batches used for culture purposes were tested for 3 weeks on the 983 cell line for growth efficiency. An optimal doubling time of about 17 hr or less is required.

For the hybridoma selection media, hypoxanthine-aminopterin-thymidine (HAT) (50X) and hypoxanthine-thymidine (HT) (50X) were used. The concentrations of aminopterin (Sigma), hypoxanthine (Sigma), and thymidine (Sigma) are 20, 5, and 0.8 mM, respectively.

Fusion was carried out using PEG (polyethylene glycol).

The concentration of the PEG solution used for fusion was 41.6% in fusion medium containing 8.75 ml dimethyl sulfoxide (Merck) added to 100 g of initial PEG solution to improve the fusion efficiency. Two-milliliter final PEG solution aliquots were sterilized by autoclaving or filtration and stored in an incubator at 37° for a maximum of 3 months.

Feeder layers of rat peritoneal cells were prepared the day before fusion in HAT medium, or 1-4 days before development or cloning in HT medium. The cells were seeded at a concentration of 2 x 10⁵ cells/ml on the basis of 0.1 ml/microwell (96-well plates) or 1 ml/macrowell (24-well plates). At no later stage, e.g., during mass culture or additional cloning, are peritoneal cells added. The origin of the rats used as the source of the feeder layers is not important and outbred Wistar rats were therefore used.

The fusion capacity of the 983 cells depends on the growth conditions. To ensure a high yield of hybrids, the IR983F cells used for the fusion must be in a strictly exponential growth phase for at least a week. The different ways to obtain this are either to add medium every day to the culture vessel (spinner or plastic flasks), adjust the cell concentration to 5 X 10⁵ cells/ml, (A. M. Lebacq-Verheyden et al, Hybridoma, 2, 355, 1983) or adjust the cell concentration back to 10⁵ cells/ml on days 0, 3, and 5.

After anesthesia, the immunized LOU rats were bled via the carotid artery. Their spleens were removed and immersed in EMEM without serum. The organ was teased under sterile conditions on a metal grid support by a beaker. The cell suspension was allowed to stand for 10 min and the large fragments that settled were discarded.

The 983 cells and the spleen cells were centrifuged for 5 min at 250xg. The pellet was washed twice with fusion medium EMEM without serum. Cells were mixed in a ratio of one 983 cell to every five spleen cells. After centrifugation (5 min at 250xg), the supernatant was discarded and the cell pellet was detached by gentle shaking. One milliliter of warmed PEG solution (37°) was added dropwise to 2 x 10⁷ cells during 90 sec of gentle shaking. After another 30 sec of shaking, 2 ml of EMEM was added during the following 90 sec to the cell mixture. Finally, 20 ml of EMEM was progressively added to dilute the PEG solution. Centrifugation for 5 min at 180 g preceded the resuspension of all cells in the selection serum-rich HAT culture medium. The cell density was adjusted to 10⁶ cells/ml, and the cell suspension was distributed over 24 (1 ml)- and 96 (0.1 ml)-well plates containing peritoneal cells in the selection HAT medium. The medium was renewed four times before starting the screening procedure.

From the seventh day after fusion, hybrid clones were observed and they were transferred to 24-well plates. When supernatants in the wells became yellow, screening for specific antibody-screting hybrid clones was performed by indirect immunofluorescence using immunizing cells as target cells. After a second screening of the whole blood, those hybrid clones which secreted antibodies reacting with lymphocytes, and not with granulocytes and/or monocytes, were selected. The hybrid lines were cloned twice by limiting dilution. The hybridoma cells were either cultured, frozen in complete DMEM-7.5% DMSO and stored in liquid nitrogen, or injected subcutaneously into LOU rats to induce solid tumors. The tumor cells were frozen in PBS- 10% FCS-7.5% DMSO and stored in liquid nitrogen, or if necessary, tumor cells were injected i.p. to induce ascitic tumor by known methods (Bazin, H. et al, J. Immunol. Methods, 71, 9, 1984). The sera or ascitic fluid of tumor-bearing rats were collected and stored at -80°C.

Among the hybrid clones secreting antibodies against the immunizing cells, most cross-reacted with granulocytes and/or monocytes and were not analyzed further. Five secreted a monoclonal antibody reacting selectively with lymphocytes, and two, designated LO-CD4-a and LO-CD4-b, secreted a monoclonal antibody that reacted with CD4. Monoclonal antibody LO-CD4-a was obtained after immunization with the T-ALL line (HPB-ALL14) and monoclonal antibody LO-CD4-b was obtained after immunization with purified T lymphocytes.

### Production and Purification of Monoclonal Antibodies LO-CD4- a and LO-CD4-b

Monoclonal antibodies LO-CD4-a and LO-CD4-b were produced in quantity either in vitro or in vivo.

For in vitro production of the monoclonal antibodies, hybridomas LO-CD4-a or LO-CD4-b were cultured under standard conditions described for fusion of the IR983F cell line essentially according to the method described by Bazin, H. ("Production of rat monoclonal antibodies with the LOU rat non-secreting 983F myeloma cell line" in Prot. Biol. Fluids, 1982, Peeters, E. ed., 9th colloquium, 1981, Pergamon Press Oxford and N.Y., pp. 615-618). The in vitro production of the monoclonal antibodies LO-CD4-a and LO-CD4-b in plates, flasks or roller bottles was conducted essentially according to the method described by Bodeus, M. et al, Immunol. Meth., 79, 1, 1985).

For in vivo production of the monoclonal antibodies, rats of the LOU/C or the LOU/C·IgK-lb(OKA) strain (Bekers, A. et al, Immunochemistry, 11, 605, 1974) were used. The rats are perfectly histocompatible with the hybridomas, LO-CD4-a and LO-CD4-b which are LOU/C hybridomas. Rats of the LOU/C·IgK-lb strain were preferred for facilitation of purification of the monoclonal antibodies. This is because LOU/C·IgK-lb rats produce Kappa light chains of the Kappa-lb allotype and monoclonal antibodies LO-CD4-a and LO-CD4-b are immunoglobulins with the IgK-la allotype light chains. Thus, contaminating IgK-lb immunoglobulins from the host can be easily eliminated by immunoadsorption on anti-IgK-lb antibodies by standard methods.

In order to adapt the hybridoma cell to in vivo culture, the first passage of in vitro-cultured hybridoma cells was subcutaneous. This route is more successful than other routes and uses a lower number of cells than is required for the intraperitoneal route.

The rat was anesthetized and 5x10⁶ hybridomas cells were injected subcutaneously in the right flank (for convenience). The rat was observed three times a week for tumor growth. The tumor generally appears after 2 or 3 weeks, but up to 3 months can elapse before the hybridoma cells begin to proliferate.

After adequate tumor growth, the rat was anesthetized and bled to check monoclonal antibody secretion. Thereafter the rat was killed, the fur swabbed with 70% ethanol, and a piece of tumor excised and disrupted PBS (0.15M phosphate buffered saline; pH 7.2) with a Potter homogenizer.

These cells were then used for ascites production.

The LOU/C rat easily develops ascites after intraperitoneal injection of syngeneic immunocytoma or hybridoma cells without any priming (Bazin, H. et al, Int. J. Cancer, 10, 568, 1972 and Bazin, H., Adv. Cancer Res., 50, 279, 1987). However, the yield of ascitic fluid can be greatly enhanced by pretreatment with a 1:1 (v:v) mixture of 2,6,10,14-tetramethylpentadecane (also called pristane®) and incomplete Freunds adjuvant (IFA). Thus ascites production can also be achieved by intraperitoneal injection of 2 ml of the mixture, called PIFA, or of IFA at the moment of tumor passage (Kints, J.-P. et al, Enhancement of ascites production by intraperitoneal injection of pristine® and incomplete Freund adjuvant. Joint meeting of Contact Group Monoclonal Antibodies and Belgian Immunological Society, ULB-VUB, Brussels, Feb. 19, 1988).

The method of producing ascites was as follows. Oil (PIFA or IFA) and cells were prepared in separate syringes in order to avoid killing the cells in the emulsion. After being anesthetized, 2 ml adjuvant was injected intraperitoneally through the right flank and cells were also injected intraperitoneally, but through the left flank to avoid accidental intrasplenic inoculation. The abdomen was gently massaged to spread the cells and oil through the whole peritoneal cavity. The rats were kept in standard conditions with food and water ad libidum. They were checked three times a week for ascitic tumor growth.

The ascites was taken when the tumor reached its maximum development. The rat was killed in an ether bell-jar and punctured with a 18G-needle. Ascites were collected in a clean flask. After swabbing with alcohol, the peritoneal cavity was opened on the left side of the abdomen. When no more liquid passed through the needle, the remaining ascitic fluid was aspirated on the side of the liver with a 20 ml syringe without a needle.

The monoclonal antibodies were purified from in vitro culture or ascites by standard ammonium sulfate procedures. Alternatively, the monoclonal antibodies were purified to homogeneity by immunoadsorption on anti-IgK-lb antibodies according to standard procedures. (See, for example, Bazin, H. et al, J. Immunol. Meth., 71, 9, 1984, for purification from ascitic fluid.)

The hybridomas that produce monoclonal antibodies LO-CD4-a and LO-CD4-b, designated LO-CD4-a and LO-CD4-b, respectively, have been deposited with the European Collection of Animal Cell Cultures and have ECACC Deposit Nos. 89012101 and 89012102, respectively.

In all Examples below, monoclonal antibody LO-CD4-a was used from a 50% saturated ammonium sulfate precipitate from ascites. Monoclonal antibody LO-CD4-b was purified to homogeneity.

### EXAMPLE 2

### REACTIVITY PATTERN OF MONOCLONAL ANTIBODIES LO-CD4-a AND LO-CD4-b WITH NORMAL CELLS

5 x 10⁵ cells in 100 »l PBS-2% new born calf serum (NBCS)-0.2% NaN₃ or 100 »l whole blood were incubated for 45 min. at 4°C with 100 »l antibody-containing supernatant or serum (1/200 dilution). Normal rat serum was used as control. Red blood cells were lysed when whole blood was used. Cells were washed once and incubated for 45 minutes at 4°C with 100 »l of rabbit anti-rat immunoglobulin (Ig) antibody F(ab′)₂ fragments labeled with fluorescein isothiocyanate (RARA-FITC), or goat anti-mouse Ig conjugated with FITC (GAM-FITC). Cells were washed once. Immunofluorescence intensity was examined either on a microscope or an EPICS cytofluorograph (Coulter).

Periphelal blood lymphocytes were isolated from blood of normal donors by a single step 1.077 density Ficoll®-Hypaque separation. Lymphocytes were activated with PHA according to known methods. Tonsil cells and thymocytes were obtained by known methods. B lymphocyte-enriched suspensions of PBL and tonsil cells were prepared by depleting the suspensions of T-lymphocytes by SRBC rosetting by known methods.

Reference monoclonal antibodies OKT3 (CD3) and OKT4 (CD4) were obtained from Ortho Diagnostics. Reference monoclonal antibodies T11 (CD2) and T12 (CD6) were purchased from Coulter. Reference monoclonal antibody LO-CD5-a (CD5) was produced by the present inventors.

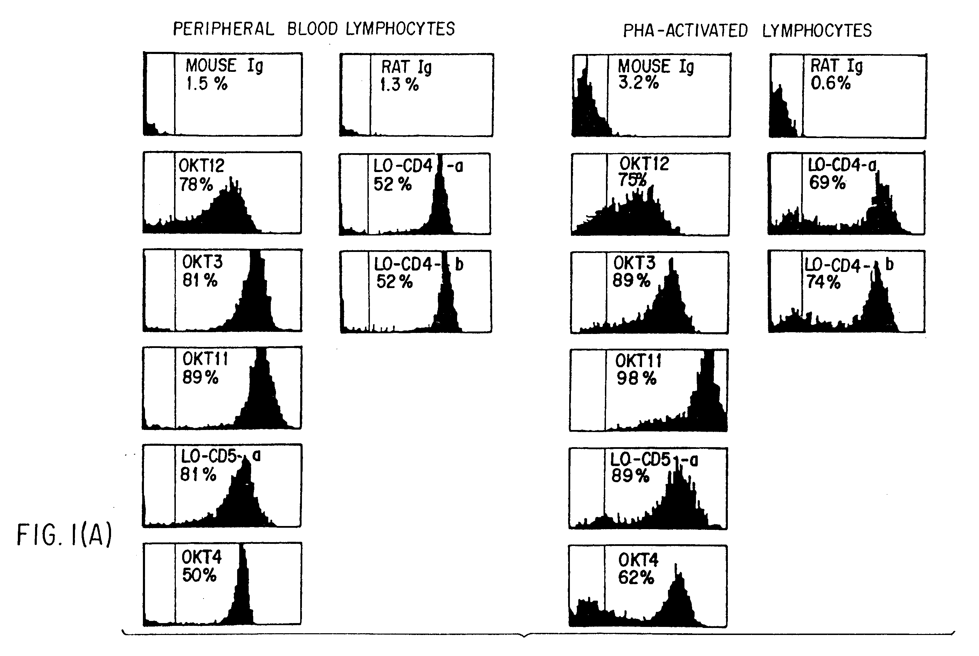

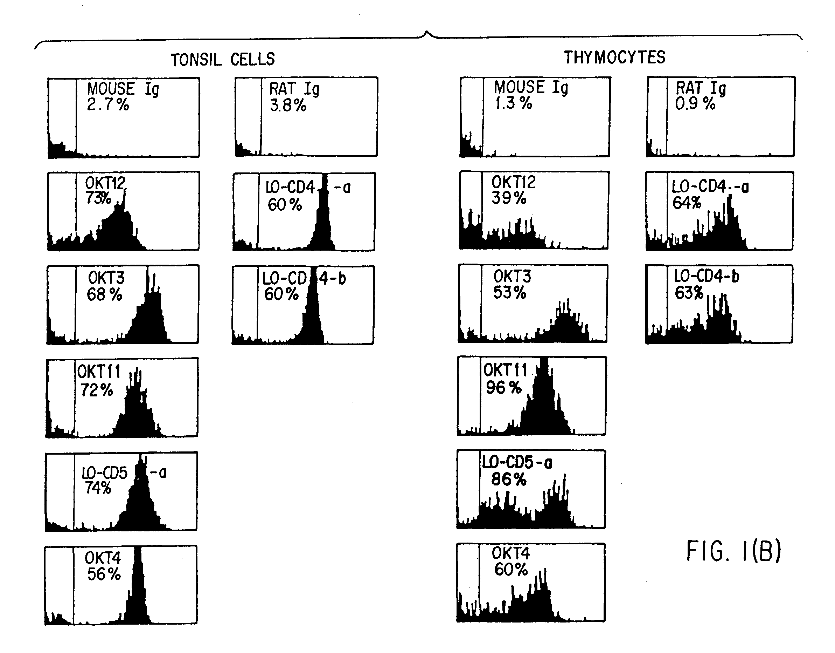

The results are shown in Table 1 and Fig. 1.

**TABLE 1**

| REACTIVITY WITH NORMAL HEMATOPOIETIC CELLS | | |
|---|---|---|
| Cells | Mabs | |
| | LO-CD4-a | LO-CD4-b |
| Blood | | |
| Lymphocytes | | |
| Total | 54% +++ | 53% +++ |
| E+ | 68% +++ | 67% +++ |
| E- | - | - |
| PHA-PBL | 69% +++ | 74% +++ |
| Monocytes | - | - |
| Granulocytes | - | - |
| RBC | - | - |
| Platelets | - | - |

| Tonsil cells | | |
|---|---|---|
| Total | 59% +++ | 60% +++ |
| E- | - | - |
| Thymocytes | 64% ++ | 66% ++ |

The monoclonal antibodies LO-CD4-a and LO-CD4-b reacted only with T lymphocytes (Table 1). No reaction was found with monocytes, granulocytes, red blood cells, platelets or with a B lymphocyte-enriched suspension prepared by depleting T lymphocytes from PBL and tonsil by SRBC rosetting. Monoclonal antibodies LO-CD4-a and LD-CD4-b reacted strongly with around 55% of PBL and about 70% of T lymphocyte-enriched PBL. Thus, these two monoclonal antibodies are specific for a subset of T lymphocytes.

Typical reactivity patterns of monoclonal antibodies LO-CD4-a and LO-CD4-b and of reference pan-T or subset-T specific monoclonal antibody OKT3, T11, T12, LO-CD5-a and OKT4 are shown in Figure 1.

In Fig. 1A, the left panel shows the reactivity patterns with PBLS and the right panel shows the reactivity patterns with PHA activated PBLs. In Fig. 1B, the left panel shows reactivity patterns with tonsil cells and the right panel shows reactivity with thymocytes. The X-axis represents fluorescence intensity and the Y-axis represents cell number.

The profiles of monoclonal antibodies LO-CD4-a and LO-CD4-b were identical to that of CD4 monoclonal antibody OKT4, whatever kind of cell was concerned. Monoclonal antibodies LO-CD4-a and LO-CD4-b reacted strongly with around 65% of thymocytes.

### EXAMPLE 3

### DOUBLE LABELING

For double immunofluorescence, tonsil cells or PBMNs were labeled with rat monoclonal antibodies and RARA-FITC as described in Example 2 above. Then the cells were incubated with phycoerythrin-conjugated monoclonal antibodies for 45 minutes at 4°C.

Tonsil cells were obtained by known methods. PBMNs from normal donors were isolated as described in Example 1. centrifugation on Ficoll®-Hypaque (d:1.077).

Reference monoclonal antibodies T11 (CD4) and B4 (CD19) were purchased from Coulter and reference monoclonal antibodies OKT4 (CD4) and OKT8 (CD8) were purchased from Ortho Diagnostics.

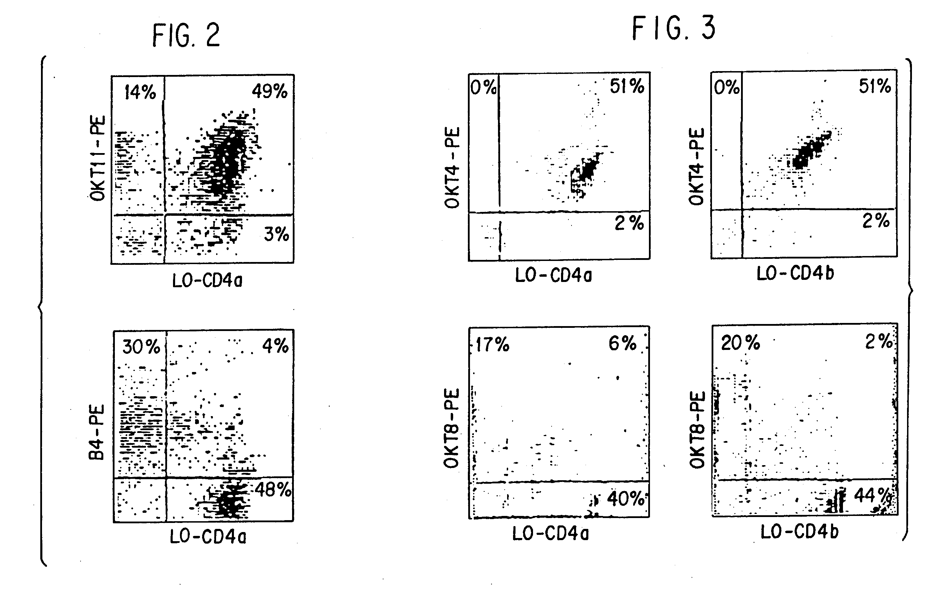

In order to confirm that LO-CD4-a and LO-CD4-b are T lymphocyte specific and to find out if the subpopulation of T lymphocytes recognized by monoclonal antibodies LO-CD4-a and LO-CD4-b is indeed the one recognized by OKT4, tonsil cells (Figure 2) or PBMN (Figure 3) were first labeled with rat monoclonal antibodies LO-CD4-a and LO-CD4-b and RARA-FITC, followed by phycoerythrin-conjugated mouse reference monoclonal antibodies T11 (CD2), B4 (CD19) (Figure 2), OKT4 (CD4) or OKT8 (CD8) (Figure 3). Cells that labeled with monoclonal antibody LO-CD4-a made up about 78% of T11+ lymphocytes (Figure 2). In contrast, the percentage of lymphocytes double-labeled with B4 and rat monoclonal antibody LO-CD4-a remained low (4-6%) (Figure 2). This weak positivity could be due to a specific binding to Fc receptors on B cells. Regarding subsets of peripheral T lymphocytes, more than 99% of T4+ lymphocytes were also labeled with LO-CD4-a or LO-CD4-b and less than 2% of PBL was labeled with OKT4 alone or with the rab monoclonal antibody alone (Figure 3). Less than 6% of PBL cells was double-labeled with OKT8 and LO-CD4-a or LO-CD4-b (Figure 3). Hence, rat monoclonal antibodies LO-CD4-a and LO-CD4-b are T lymphocyte specific. Further, the subpopulation of T lymphocytes recognized by LO-CD4-a and LO-CD4-b is exactly that recognized by OKT4, not by OKT8 (T4+, T8-).

### EXAMPLE 4

### REACTIVITY PATTERNS OF LO-CD4-a AND LO-CD4-b WITH LEUKEMIC CELLS

In order to detect cross-reactions with immature B or myeloid cells and to delimit at which stage of differentiation the different T antigens appear or disappear, leukemias, lymphomas (Table 2) and the leukemia-derived cell lines (Tables 3 and 4) were studied.

Indirect immunofluorescence was performed as described in Example 2.

Leukemia and lymphoma cells T-ALL, T-CLL, B-CLL, B-NHL and CALL were obtained from leukemic patients by known methods. Leukemia-derived cell lines of B lineage (Nalm-1, KM3, Nalm-6, Raji and Daudi), Myeloid line (U937, HL-60 and K562) and CCRF-CEM, HPB-ALL, MOLT-4 and JURKAT are all publicly available.

HPB-ALL, CCRF-CEM, MOLT-4, JURKAT, Raji, Daudi, Nalm-1, Nalm-6, KM3, U937, K562 and HL-60 were cultured at 37°C in the presence of 5% CO₂ in RPMI-1640 medium containing 10% fetal calf serum, 2 mM glutamine and PS.

Monoclonal antibodies OKT3 (CD3) and OKT4 (CD4) were purchased from Ortho Diagnostics. Monoclonal antibodies T11 (CD2) and T12 (CD6) were purchased from Coulter. Monoclonal antibody Leu3a was purchased from Becton-Dickenson. Monoclonal antibody LO-CD5-a (CD5) was produced by the present inventors and used as an example of an anti-CD5 monoclonal antibody.

**TABLE 2**

| REACTIVITY WITH LEUKEMIA CELLS | | |
|---|---|---|
| Cells | Mabs | |
| | LO-CD4-a | LO-CD4-b |
| T-ALL | | |
| 1 | 94% +++ | 97% +++ |
| 2 | 56% +++ | 64% + |
| 3 | 24% ± | 24% ± |
| 4 | 20% ± | 19% ± |
| 5 | 89% ++ | 89% ++ |
| 6 | 54% ++ | 61% ++ |
| T-CLL | 94% ++ | 93% ++ |

| B-CLL | | |
|---|---|---|
| 1 | 3% ++ | 5% ++ |
| 2 | 4% ++ | 4% ++ |
| 3 | 2% ++ | 3% ++ |
| 4 | - | - |
| B-NHL | 4% + | 5% + |

| cALL | | |
|---|---|---|
| 1 | 8% ++ | 7% ++ |
| 2 | 2% + | 2% + |
| 3 | - | - |

**TABLE 3**

| REACTIVITY WITH NON-T CELL LINES | | |
|---|---|---|
| Cells | Mabs | |
| | LO-CD4-a | LO-CD4-b |
| B lineage | | |
| Nalm-1 | - | - |
| KM3 | - | - |
| Nalm-6 | - | - |
| Raji | - | - |
| Daudi | - | - |

| Myeloid lines | | |
|---|---|---|
| U937 | ++ | ++ |
| HL-60 | ± | ± |
| K562 | - | - |

**TABLE 4**

| REACTIVITY WITH T CELL LINES | | | | |
|---|---|---|---|---|
| Monoclonal Antibodies | CEM | HPB-ALL | MOLT-4 | JURKAT |
| OKT3 | - | ++ | - | ± |
| T11 | +++ | +++ | +++ | ++ |
| T12 | + | + | + | - |
| LO-CD5-a | ++ | ++ | ++ | ± |
| Leu3a | +++ | +++ | ± | - |
| OKT4 | +++ | +++ | ± | - |
| LO-CD4-a | +++ | +++ | ± | - |
| LO-CD4-b | +++ | +++ | ± | - |

No cross-reaction of monoclonal antibodies LO-CD4a and LD-CD4-b with non-T cell lines, listed in Table 3, was found, except that with U937 just as OKT4 and Leu3a did (data not shown). This cell line is known to express the T4 antigen.

Non-T non-B lymphoblasts were not labeled by monoclonal antibody LO-CD4-a or LO-CD4-b. (Table 2). The detection of 2-12% positive cells can be assigned to the presence of residual normal lymphocytes. The 2-10% cells brightly labeled with LO-CD4-a and LO-CD4-b are probably due to residual normal cells. LO-CD-4-a and LO-CD4-b stained all T-ALL cell lines tested (Table 2) with variable intensity as did OKT4 (data not shown).

Table 4 shows that the LO-CD4-a and LO-CD4-b antigen is present on CEM and HPB-ALL T-cell lines, weakly expressed on the MOLT-4 T-cell line and not expressed on the JURKAT T-cell line. This is the same pattern exhibited by monoclonal antibodies Leu3a and OKT4.

### EXAMPLE 5

### INHIBITION OF OKT4 AND Leu3a BINDING

Monoclonal antibodies LO-CD4-a and LO-CD4-b recognized exactly the same subset of T lymphocytes as OKT4 (Figure 3). Therefore blocking of binding of mouse CD4 monoclonal antibodies by LO-CD4-a and LO-CD4-b was investigated to determine if LO-CD4-a and LO-CD4-b compete for the same epitopes as either OKT4 or Leu3a.

Lymphocytes were isolated from peripheral blood by a single step 1.077 density Ficoll-Hypaque separation (PBLs). Mouse monoclonal antibody OKT4 (CD4) was purchased from Ortho Diagnostics and monoclonal antibody Leu3a (CD4) was purchased from Becton-Dickinson. Mouse-anti rat monoclonal antibody was obtained from Jackson Immunoresearch Laboratories. OKT4 mouse anti-CD4 FITC conjugate (OKT4FITC) was purchased from Ortho Diagnostics, Leu3a mouse anti-CD4 phycoerythrin conjugate (Leu3aPE) was purchased from Bectin-Dickenson, and mouse anti-rat FITC conjugate (MARFITC) was purchased from Jackson, Immunoresearchb Laboratories.

The PBL concentration was adjusted to 1 x 10⁷ cells/ml in Assay Buffer (AB) (1% BSA, 0.1% NaN₃, 6»M colchicine in PBS, pH 7.4). Monoclonal antibodies LO-CD4-a and LO-CD4-b were diluted to 100 »g/ml protein in AB. Then 100 »l of cell suspension was incubated with 10 »l of either diluted rat monoclonal antibody LO-CD4-a or LO-CD4-b for 30 min. at 4°C with-occasional agitation. The cells were washed by resuspending in 2.0 ml of fiB and then centrifuged at 400xg for 5 min. Washing and centrifugation was repeated two more times. Next the washed cells in 100 »l AB were incubated with 10 »l (100 »g/ml protein) of MARFITC, OKT4FITC, or Leu3aPE for 30 min. at 4°C with occasional agitation. Alternatively, the cells were incubated with 10 »l of OKT4FITC or Leu3aPE without preincubation with monoclonal antibodies LO-CD4-a or LO-CD4-b. The cells were washed twice as described above and fixed in 0.8 ml of formalin in PBS. The cells were analyzed on a Becton Dickinson FACScan flow cytometer.

The data are shown in Figure 4.

In Figure 4, the X-axis in all panels represents green fluorescence (Fluorescence 1) or red fluorescence (Fluorescence 2) and the Y-axis in all panels represents counts full scale. Figs. 4(A) to 4(G) show the data for monoclonal antibody LO-CD4-a and Figs. 4(A) to 4(N) show the data for monoclonal antibodies LO-CD4-b.

Figs. 4(A) and 4(B) are controls showing green and red autofluorescence for PBLs in the absence of added antibody conjugate, respectively. Fig. 4(C) is a control showing the binding of monoclonal antibody LO-CD4-a to PBLs detected by MARFITC. Fig. 4(D) is a control showing the fluorescence intensity of PBLs after incubation with OKT4FITC only. Fig. 4(E) shows the fluorescence intensity of PBLs incubated with OKT4FITC after preincubation with LO-CD4-a. Fig. 4(F) is a control showing the fluorescence intensity of PBLs after incubation with Leu3apE only. Fig. 4(G) shows the fluorescence intensity of PBLs incubated with Leu3aPE after preincubation with LO-CD4-a.

Figs. 4(H) to 4(N) parallel Figs. 4(A) to 4(G), except that where monoclonal antibody LO-CD4-a was used in Figs. 4(A) to 4(G), monoclonal antibody LO-CD4-b was used in Figs. 4(H) to 4(N).

The data in Figure 4 is summarized in Table 5 below.

**TABLE 5**

| EFFECT OF MONOCLONAL ANTIBODIES LO-CD4-a and LO-CD4-b ON BINDING OF OKT4 AND Leu3a TO PBLS | |
|---|---|
| | Relative Fluorescence Intensity |
| OKT4 FITC only | 100 |
| OKT4FITC after preinc. with LO-CD4-a | 97 |
| Leu3aPE only | 100 |
| Leu3aPE after preinc. with LO-CD4-a | 99 |
| OKT4FITC only | 100 |
| OKT4FITC after preinc. with LO-CD4-b | 99.6 |
| Leu3aPE only | 100 |
| Leu3APE after preinc. with LO-CD4-b | 99 |

From Fig. 4 and Table 5 it is apparent that preincubation with either LO-CD4-a or LO-CD4-b does not reduce the Relative Fluorescence Intensity for either OKT4FITC or Leu3aPE. Thus, LO-CD4-a and LO-CD4-b do not compete for either of the epitopes for which OKT4 and Leu3a are specific.

### EXAMPLE 6

### BINDING OF MONOCLONAL ANTIBODIES LO-CD4-a AND LO-CD4-b TO CD4 POSITIVE DEPLETED PBLs

This Example demonstrates that monoclonal antibodies LO-CD4-a and LO-CD4-b bind to the same lymphocyte subpopulation as OKT4.

Lymphocytes were isolated from peripheral blood by a single step 1.077 density Ficoll®-Hypaque separation (PBLs).

Goat anti-rat monoclonal antibody was obtained from Jackson Research Laboratories. Mouse monoclonal antibody OKT4 (CD4) and OKT8 (CD8) were purchased from Ortho Diagnostics. Mouse monoclonal antibody Leu3 (CD4) was purchased from Becton-Dickinson. Goat anti-rat phycoerythrin conjugate (GARPE), monoclonal antibody OKT4FITC, OKT8FITC and Leu3aPE were prepared according to conventional methods. Sheep anti-mouse coated magnetic microparticles (SAM Beads) were obtained from DYNAL INC.

The PBL concentration was adjusted to 1x10⁷ cells/ml with AB (see Example 5). 2x10⁷ cells were incubated with 200 »l (100 »g/ml) OKT4 for 30 min. at 4°C with occasional agitation. The cells were washed by resuspending in 2 ml of AB and then centrifuged at 400xg for 5 min. Washing and centrifugation was repeated two more times. The cells were resuspended in 1 ml of AB and incubated with SAM Beads (40:1 beads per cell) for 30 min. at 4°C with agitating every 5 min. A magnetic field was then applied for 1 min. and nonadherent cells were removed. The nonadherent cells were washed twice with AB and the concentration adjusted to 1x10⁷ cells/ml with AB.

The CD4 depleted cell population was then immunofluorescently stained for OKT4, Leu3a, OKT8, LO-CD4-a and LO-CD4-b by conventional methods, and analyzed on a Becton Dickinson FACScan flow cytometer.

Alternatively, undepleted cells were adjusted to 1x10⁷ cells/ml with AB, washed as described in Example 5 and immunofluroscently stained for OKT4, Leu3a, OKT8, LO-CD4-a and LO-CD4-b by conventional methods, and then analyzed on a Becton Dickinson FACSCan flow cytometer.

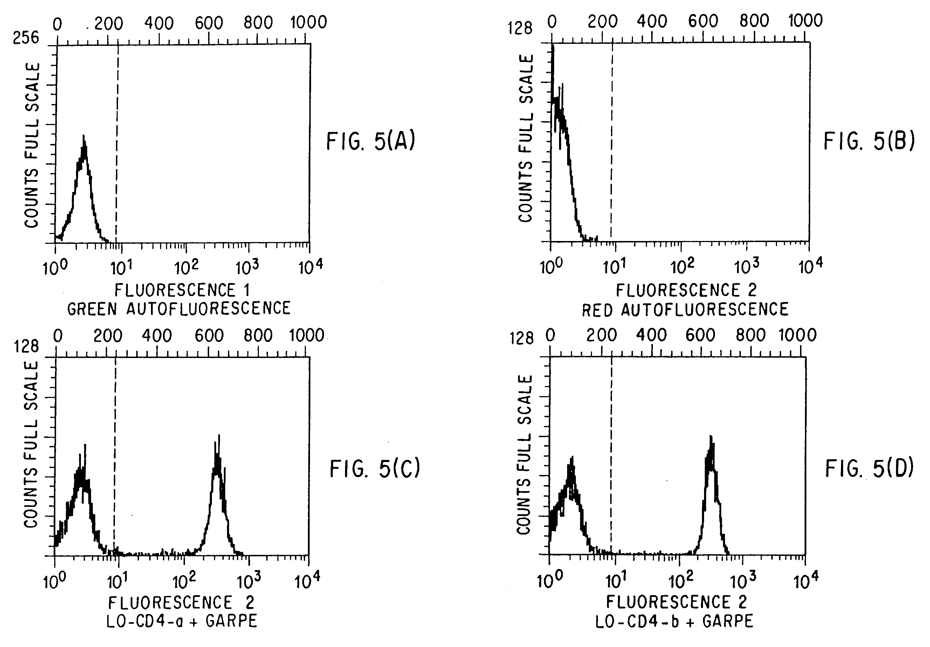

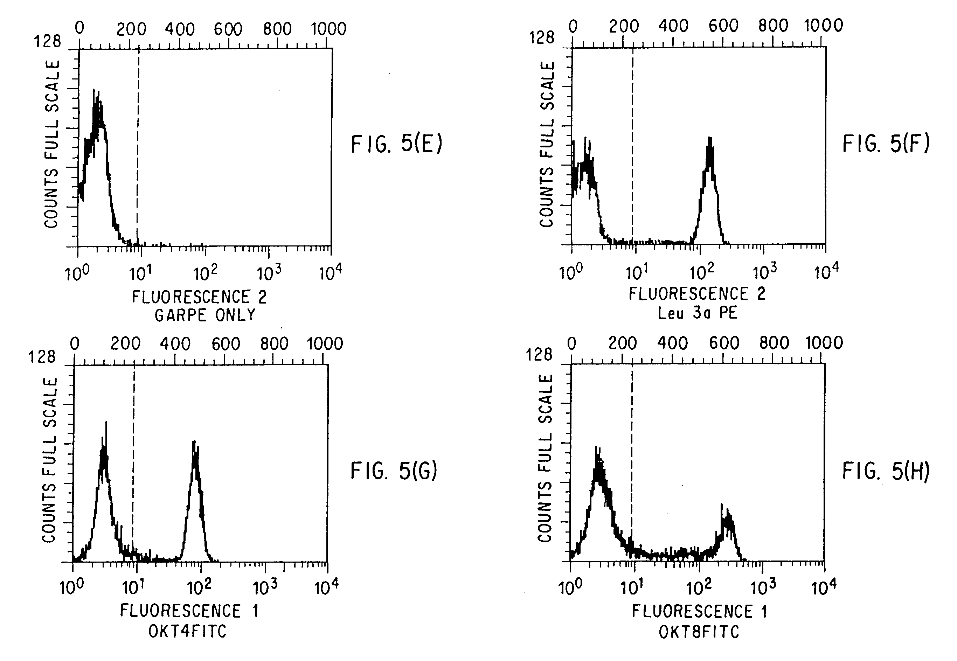

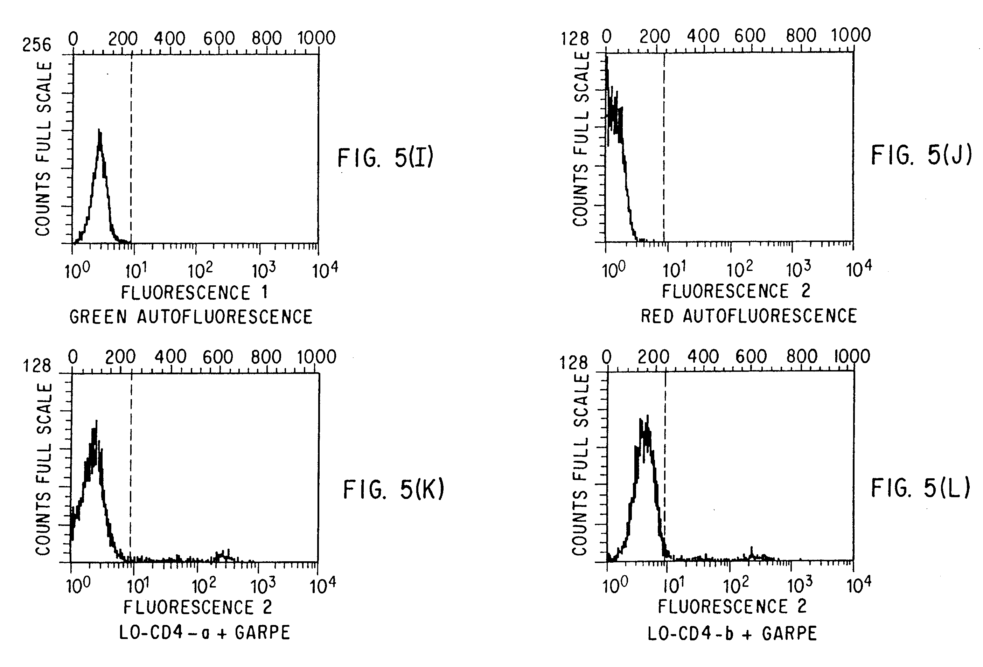

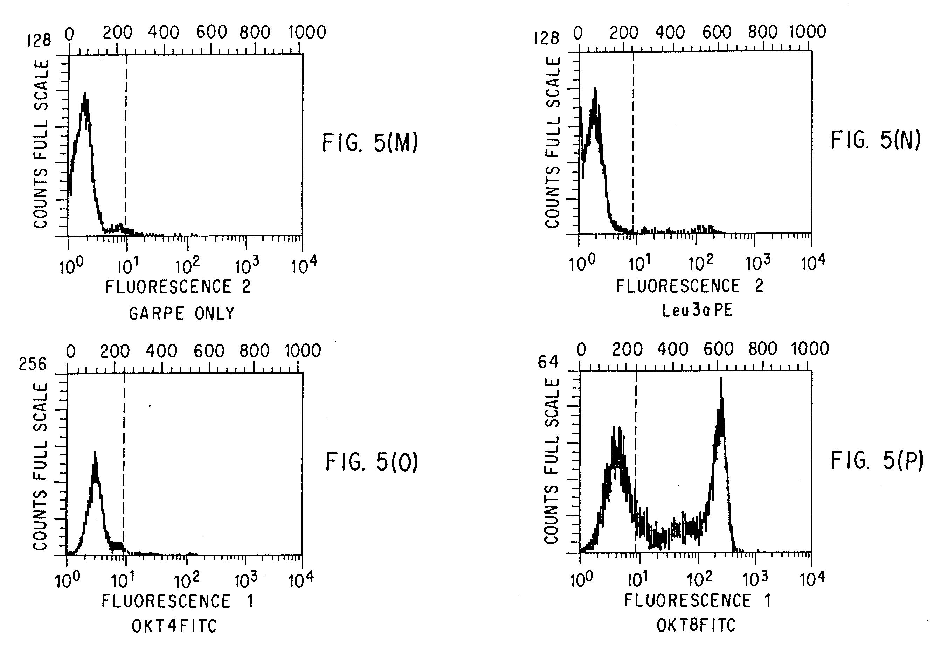

The data are shown in Figure 5.

In Fig. 5, the X-axis in all panels represents green fluorescence (Fluorescence 1) or red fluorescence (Fluorescence 2) and the Y-axis in all panels represents counts full scale.

Figs. 5(A) to 5(H) show the data for undepleted PBLs and Figs. 5(I) to 5(P) show the data for CD4 depleted PBLs.

Figs. 5(A) and 5(B) are controls showing green and red autofluorescence, respectively, for PBLs in the absence of added antibody conjugate. Figs. 5(C) and 5(D) show binding of monoclonal antibodies LO-CD4-a and LO-CD4-b, respectively, to undepleted PBLs detected by GARPE. Fig. 5(E) is a control showing immunofluorescence of PBLs after incubation with GARPE. Figs. 5(F), 5(G) and 5(H) show the fluorescence intensity of undepleted PBLs after incubation with Leu3aPE, OKT4FITC and OKT8FITC, respectively.

Figs. 5(I) and 5(P) parallel Figs. 5(A) to 5(H), except that depleted PBLs were used in place of undepleted PBLs.

The data in Figure 5 is summarized in Table 6 below.

**TABLE 6**

| BINDING OF MONOCLONAL ANTIBODIES LO-CD4-a AND LO-CD4-b TO UNDEPLETED AND DEPLETED PBLs | |
|---|---|
| Undepleted PBLs | % PBLs Labeled |
| OKT4 positive | 44 |
| Leu3a positive | 39 |
| OKT8 positive | 31 |
| LO-CD4-a positive | 41 |
| LO-CD4-b positive | 42 |

| CD4 Depleted PBLs | |
|---|---|
| OKT4 positive | 2 |
| Leu3a positive | 5 |
| OKT8 positive | 58 |
| LO-CD4-a positive | 7 |
| LO-CD4-b positive | 8 |

From Fig. 5 and Table 6 it is apparent that depletion of CD4 positive lymphocytes from PBLs was nearly complete with only 2% OKT4 positive cells and 5% Leu3a remaining. Also, both LO-CD4-a and LO-CD4-b positive lymphocytes were significantly reduced in the depleted PBL population. This data indicates that both LO-CD4-a and LO-CD4-b rat monoclonal antibodies are indeed specific for the same lymphocyte subpopulation as OKT4.

### EXAMPLE 7

### TWO COLOR FLUORESCENCE PBL BINDING ASSAY

This Example shows that monoclonal antibodies LO-CD4-a and LO-CD4-b bind exclusively to CD4 positive lymphocytes.

Lymphocytes were isolated from peripheral blood by a single step 1.077 density Ficoll®-Hypaque separation (PBLs).

Mouse Leu3A (CD4) was purchased from Becton-Dickenson. Mouse-anti-rat monoclonal antibody was obtained from Jackson Immunoresearch Laboratories. Leu3aPE was purchased from Bectin-Dickenson and MARFITC was purchased from Jackson Immunoresearch Laboratories.

The PBL concentration was adjusted to 1x10⁷ cells/ml in AB (see Example 5). 100 »l of cell suspension was incubated with 10 »l (100 ug/ml) of Leu3aPE for 30 min. at 4°C with occasional agitation. The cells were washed by resuspending in 2 ml of AB and then centrifuged at 400xg for 5 min. Washing and centrifugation was repeated two more times. Cells were resuspended in 100 »l of AB. Monoclonal antibodies LO-CD4-a and LO-CD4-b were diluted to 100 »g/ml in AB. The washed cells were resuspended in 100 »l AB and incubated with 10 »l of diluted antibody for 30 min. at 4°C with occasional agitation. The cells were washed twice with AB and resuspended in 100 »l of AB MARFITC was then diluted 1:10 (v:v) (100 »g/ml) in AB. 10 »l of diluted MARFITC was then added to the 100 »l of washed cells and incubated for 20 min. at 4°C with occasional agitation. Alternatively, before adding MARFITC, the cells (1x10⁷ in 100 »l AB) were incubated with Leu3aPE 10»l (100 »g/ml) and washed, as described above. The incubation mixture was centrifuged and washed twice with AB as described above and fixed in 0.8 ml 1% formalin in PBS. The cells were analyzed on a Becton Dickinson FACScan flow cytometer.

Dot plot analyses of the flow cytometry data of red fluorescence vs. green fluorescence was then carried out. The results were shown in Fig. 6, where the X-axis of each plot represents green fluorescence (Fluorescence 1) and the Y-axis of each plot represents red fluorescence (Fluorescence 2).

In the dot plot analysis, those cells which remain unbound by either Leu3a or the LO-CD-4 rat monoclonal antibodies are represented in the third quadrant (see Figs. 6(A) and 6(E)). These cells have the least amount of fluorescent character. Cells bound by Leu3aPE are represented in the first quadrant as having red fluorescent character and those cells bound by either rat anti-CD4 monoclonal are represented in the fourth quadrant as having green fluorescent character. The particular cell population that binds both the Leu3a and the rat anti-CD4 monoclonals are represented in quadrant 2 having both green and red fluorescent character. If either LoCD4 rat monoclonal exclusively binds CD4 positive cells, quadrants 2 and 3 will be the only quadrants occupied. If either quadrants 1 and/or 4 are occupied as well as quadrant 2, this indicates that the immunoglobulin is not exclusively specific for CD4.

Figs. 6(A) to 6(D) show the data for monoclonal antibody LO-CD4-b, and Figs. 6(E) to 6(I) show the data for monoclonal antibody LO-CD4-a.

Fig. 6(A) is a control showing the dot plot for red and green autofluorescence of PBLs in the absence of added antibody conjugate. Fig. 6(B) shows the dot plot for fluorescence of PBLs incubated with monoclonal antibody Leu3aPE. Fig. 6(C) shows the dot plot for fluorescence of PBLs incubated with MARFITC after preincubation with monoclonal antibody CO-CD4-b. Fig. 6(D) shows the dot plot for fluorescence of PBLs incubated with monoclonal Leu3PE and then MARFITC after preincubation with LO-CD4-b.

Figs. 6(E) to 6(H) parallel Figs. 6(A) to 6(D), except that monoclonal antibody LO-CD4-a was used instead of monoclonal antibody LO-CD4-b. Fig. 6(I) shows the dot plot for fluorescence of PBLs incubated with MARFITC after preincubation with Leu3a.

The data from figs. 6(A) to 6(D) and from Figs. 6(E) to 6(I) is summarized in Tables 7 and 8, respectively, below.

**TABLE 7**

| LO-CD4-b TWO COLOR ASSAY | | | | |
|---|---|---|---|---|
| | Quad 1 (red) | Quad 2 (green) | Quad 3 (auto) | Quad 4 (red & green) |
| Leu3a + | 48.3% | 0.5% | 51.0% | 0.2% |
| LO-CD4-b + | 0.0% | 0.2% | 52.4% | 47.6% |
| Two Color | 1.2% | 47.1% | 51.5% | 0.2% |

**TABLE 8**

| LO-CD4-a TWO COLOR ASSAY | | | | |
|---|---|---|---|---|
| | Quad 1 (red) | Quad 2 (green) | Quad 3 (auto) | Quad 4 (red & green) |
| Leu3a + | 41.2% | 0.1% | 58.7% | 0.0% |
| LO-CD4-a + | 0.1% | 0.2% | 58.1% | 41.6% |
| Two Color | 1.8% | 40.4% | 57.6% | 0.0% |

The data from this Example indicates that both LO-CD4-a and LO-CD4-b are specific for only CD4 positive PBLs. Greater than 96% of Leu3a positive lymphocytes became double labelled by either LO-CD4-a or LO-CD4-b. No other PBL population was double label led by LO-CD4-a or LO-CD4-b.

### EXAMPLE 8

### INDIRECT BINDING ASSAY OF MONOCLONAL ANTIBODIES LO-CD4-a AND LO-CD4-b TO PERIPHERAL BLOOD LYMPHOCYTES

This Example establishes binding patterns of monoclonal antibodies LO-CD4-a and LO-CD4-b to peripheral blood lymphocytes (PBLs).

The lymphocyte population was obtained from veneous peripheral blood by a single step 1.077 density Ficoll®-Hypaque separation.

Monoclonal antibodies OKT4 (CD4) and OKT8 (CD8) were purchased from ortho Diagnostics and Leu3a (CD4) was purchased from Becton Dickinson. Goat anti-rat phycoerythrin conjugate (GARPE) and goat anti-mouse phycoerythrin conjugate (GAMPE) were obtained from Jackson Research Laboratories.

The PBL concentration was adjusted to 1x10⁷ cells/ml in AB (see Example 5). Rat monoclonal antibodies LO-CD4-a and LO-CD4-b and mouse monoclonal antibodies OKT4, OKT8 and Leu3a were used as received from the vendor. 10 »l of the monoclonal antibodies was added to 100 »l of the cell suspension and incubated for 30 min. at 4°C with occasional agitation. The cells were washed by resuspending in 2 ml of AB and then centrifuged at 400 xg for 5 min. Washing and centrifugation was repeated two more times. Cells were resuspended in 100 »l of AB. The conjugate GARPE or GAMPE was diluted 1:10 (v:v) (100 »g/ml) in AB. 10 »l of the appropriate diluted conjugate was added to the 100 »l of washed cells and incubated for 20 min. at 4°C with occasional agitation. The cells were washed twice as described above and fixed in 0.8 ml 1% formalin in PBS. The cells were analyzed on a Becton Dickinson FACScan flow cytometer.

The data are shown in Figure 7.

In Fig. 7, the X-axis in all panels represents green fluorescence (Fluorescence 1) or red fluorescence (Fluorescence 2) and the Y-axis in all panels represents counts full scale. Figs. 7(A) and 7(B) are controls showing green and red autofluorescence, respectively, for PBLs in the absence of added antibody conjugate. Fig. 4(C) is a control showing the fluorescence for PBLs incubated only with GARPE. Fig. 7(D) shows the fluorescence intensity of PBLs incubated with OKT8 as detected by GAMPE. Figs. 7(E) and 7(F) show the fluorescence intensity of PBLs incubated with OKT4 and Leu3a, respectively, as detected by GAMPE. Figs. 7(G) and 7(H) show the fluorescence intensity of PBLs incubated with LO-CD4-a and LO-CD4-b, respectively, as detected by GARPE.

The data in Fig. 7 are summarized in Table 9 below.

**TABLE 9**

| INDIRECT BINDING ASSAY OF LD-CD4-a AND LD-CD4-b TO PBLs | |
|---|---|
| Ab | % PBLs Bound |
| GARPE | 0 |
| OKT4 | 44 |
| Leu3a | 39 |
| LO-CD4-a | 42 |
| LO-CD4-b | 42 |

From the results in Fig. 7 and Table 9, it is evident that LO-CD4-a and LO-CD4-b are not "pan lymphocyte" antibodies because both bind less than 50% of a PBL population.

### EXAMPLE 9

### FUNCTIONAL ANALYSIS OF LO-CD4-a AND LO-CD4-b

In order to find out whether the antigens defined by LO-CD4-a and LO-CD4-b have any functional significance in immune response, the effect of these monoclonal antibodies on lymphocyte proliferation induced by lectins, antigens or OKT3 was studied.

For all experiments, cells were cultured at 37°C in the presence of 5% CO₂ in 96-well plates containing 200 »l RPMI-1640 medium supplemented with 20% human AB serum, 2 mM glutamine, 100 U/ml penicillin, 100 »g/ml streptomycin and 25 mM Hepes. 10⁵ peripheral blood mononuclear cells (PBMN) were cultured with 2 »g/ml PHA, 20 »g/ml ConA, 0.5 »g/ml T3 or 10 »g/ml pokeweed mitogen (PWM) or 20 mU/ml tetanus toxoid (TT) in the absence or the presence of rat monoclonal antibody serum diluted at 1/500. The PBMNs were isolated as described in Example 1. For the allogeneic mixed lymphocyte reaction, 105 PBMN from two individual donors were cultured together in the presence or absence of rat monoclonal antibody. 3H-thymidine (3H-TdR, 6.5 Ci/mmol, New England Nuclear) was added to each well (5 »Ci/well) 3 days after stimulation with ConA, PHA, PWM, or T3, or 5 days after stimulation with TT or in MLR and incubation was continued overnight. The cells were harvested with a cell harvestor (SKATRON) and 3H-TdR incorporation was measured in a scintillation counter.

The results are shown in Table 10 below.

**TABLE 10**

| EFFECT OF MONOCLONAL ANTIBODIES LO-CD4-a AND LO-CD4-b ON LYMPHOCYTE PROLIFERATION INDUCED BY LECTINS, OKT3 OR ANTIGENS | | | |
|---|---|---|---|
| Monoclonal Antibody | Percentage of Control | | |
| | PHA | ConA | PWM |
| Diluent Control | 100 | 100 | 100 |
| LO-CD4-a | 106±4 | 88±2 | 90±6 |
| LO-CD4-b | 116±6 | 59±16 | 72±1 |

| Monoclonal Antibody | Percentage of Control | | |
|---|---|---|---|
| | T3 | TT | MLR |
| Diluent Control | 100 | 100 | 100 |
| LO-CD4-a | 92±9 | 43±4 | 67±13 |
| LO-CD4-b | 73±9 | 32±5 | 34±9 |

Results are expressed as mean ± standard deviation.

From Table 10, it is apparent that the effects of monoclonal antibodies LO-CD4-a and LO-CD4-b on lymphocyte proliferation are quantitatively different, LO-CD4-b always having stronger inhibitive effect than LO-CD4-a. The monoclonal antibodies interfered strongly with proliferation of lymphocytes induced by the soluble antigen tetanus toxoid (TT) and by cell-bound antigen (MLR): About 70% inhibition by LO-CD4-b and 40-60% inhibition by LO-CD4-a was observed. Inhibition of ConA, PWM and T3-induced proliferation was weak (30-40%) by LO-CD4-b, and was not significant by LO-CD4-a. Neither monoclonal antibody had a suppressive effect on PHA-induced proliferation.

## Claims (Claims for the following Contracting State(s): AT, BE, CH, LI, DE, FR, GB, IT, NL)

1. A method of producing rat monoclonal antibodies that bind to human CD4 antigen, comprising the steps of:
(1) immunizing a rat or rat immunocompetent cells in vitro with an immunogen comprising CD4 antigen;
(2) fusing immunized cells from said rat or immunized rat immunocompetent cells with immunocytoma cells;
(3) selecting hybridoma cells that produce antibody that binds to a human CD4 antigen epitope distinct enough from that bound by murine monoclonals so there is no cross blocking;
(4) culturing the selected hybridoma cells; and
(5) recovering said antibody.

2. The method of Claim 1, wherein said immunogen is a T-cell line that expresses CD4 antigen or purified T lymphocytes.

3. The method of Claim 1, wherein said immunocytoma cells are rat or mouse immunocytoma cells.

4. A hybridoma that produces a rat monoclonal antibody that binds to a human CD4 antigen epitope distinct enough from that bound by murine monoclonals so there is no cross blocking.

5. The hybridoma of Claim 4, wherein said rat monoclonal antibody binds to a human CD4 antigen epitope other than those epitopes defined by murine monoclonal antibodies OKT4 and Leu3a.

6. A hybridoma that produces antibodies having the binding specificity of antibodies produced by hybridoma LO-CD4-a having ECACC Deposit No. 89012101.

7. A hybridoma that produces antibodies having the binding specificity of antibodies produced by hybridoma LO-CD4-b having ECACC Deposit No. 89012102.

8. A rat monoclonal antibody that binds to a human CD4 antigen epitope distinct enough from that bound by murine monoclonals so there is no cross blocking.

9. The rat monoclonal antibody of Claim 8, wherein said monoclonal antibody binds to a human CD4 antigen epitope other than those epitopes defined by murine monoclonal antibodies OKT4 and Leu3a.

10. A rat monoclonal antibody having the binding specificity of monoclonal antibody LO-CD4-a produced by hybridoma LO-CD4-a having ECACC Deposit No. 89012101.

11. A rat monoclonal antibody having the binding specificity of monoclonal antibody LO-CD4-b produced by hybridoma LO-CD4-b having ECACC Deposit No. 89012102.

12. A binding protein having the binding specificity of monoclonal antibody LO-CD4-a produced by hybridoma LO-CD4-a having ECACC Deposit No. 89012101.

13. A binding protein having the binding specificity of monoclonal antibody LO-CD4-b produced by hybridoma LO-CD4-b having ECACC Deposit No. 89012102.

14. Use of a rat monoclonal antibody according to any of Claims 8 to 13 for manufacturing a preparation for an in vivo therapeutic method.

15. An in vitro diagnostic method comprising contacting a rat monoclonal antibody produced according to any of Claims 1 to 13 with a suitable test sample and assaying for the CD4 antigen.

## Claims (Claims for the following Contracting State(s): ES)

1. A method of producing rat monoclonal antibodies that bind to human CD4 antigen, comprising the steps of:
(1) immunizing a rat or rat immunocompetent cells in vitro with an immunogen comprising CD4 antigen;
(2) fusing immunized cells from said rat or immunized rat immunocompetent cells with immunocytoma cells;
(3) selecting hybridoma cells that produce antibody that binds to a human CD4 antigen epitope distinct enough from that bound by murine monoclonals so there is no cross blocking;
(4) culturing the selected hybridoma cells; and
(5) recovering said antibody.

2. The method of Claim 1, wherein said immunogen is a T-cell line that expresses CD4 antigen or purified T lymphocytes.

3. The method of Claim 1, wherein said immunocytoma cells are rat or mouse immunocytoma cells.

4. The method of Claim 1, wherein said monoclonal antibody binds to a human CD4 antigen epitope other than those epitopes defined by murine monoclonal antibodies OKT4 and Leu3a.

5. The method of Claim 1, wherein said monoclonal antibody has the binding specificity of monoclonal antibody LO-CD4-a produced by hybridoma LO-CD4-a having ECACC Deposit No. 89012101.

6. The method of Claim 1, wherein said monoclonal antibody has the binding specificity of monoclonal antibody LO-CD4-b produced by hybridoma LO-CD4-b having ECACC Deposit No. 89012102.

7. A method of producing a hybridoma that produces a rat monoclonal antibody that binds to human CD4 antigen, comprising the steps of:
(1) immunizing a rat or rat immunocompetent cells in vitro with an immunogen comprising CD4 antigen;
(2) fusing immunized cells from said rat or immunized rat immunocompetent cells with immunocytoma cells;
(3) selecting hybridoma cells that produce antibody that binds to a human CD4 antigen epitope distinct enough from that bound by murine monoclonals so there is no cross blocking.

8. The method of Claim 7, wherein said immunogen is a T-cell line that expresses CD4 antigen or purified T lymphocytes.

9. The method of Claim 7, wherein said immunocytoma cells are rat or mouse immunocytoma cells.

10. The method of Claim 7, wherein said rat monoclonal antibody binds to a human CD4 antigen epitope other than those epitopes defined by murine monoclonal antibodies OKT4 and Leu3a.

11. The method of Claim 7, wherein said monoclonal antibody has the binding specificity of antibodies produced by hybridoma LO-CD4-a having ECACC Deposit No. 89012101.

12. The method of Claim 7, wherein said monoclonal antibody has the binding specificity of antibodies produced by hybridoma LO-CD4-b having ECACC Deposit No. 89012102.

13. An in vitro diagnostic method comprising contacting a rat monoclonal antibody produced according to any of Claims 1 to 6 with a suitable test sample and assaying for the CD4 antigen.

## Patentansprüche (Patentansprüche für folgende(n) Vertragsstaat(en): AT, BE, CH, LI, DE, FR, GB, IT, NL)

1. Verfahren zur Herstellung monoklonaler Antikörper der Ratte, die an CD4-Humanantigen binden, welches folgende Schritte umfaßt:
(1) Immunisieren einer Ratte oder von immunokompetenten Rattenzellen in vitro mit einem Immunogen, welches CD4-Antigen umfaßt,
(2) Verschmelzen der immunisierten Zellen der Ratte oder der immunisierten immunokompetenten Rattenzellen mit Immunocytomzellen,
(3) Selektieren der Hybridzellen, die Antikörper produzieren, die an ein CD4-Humanantigenepitop binden, welches ausreichend von demjenigen Epitop abgesetzt ist, an das von Mäusen abgeleitete monoklonale Antikörper binden, daß, es nicht zur Kreuzblockierung kommt,
(4) Züchten der selektierten Hybridzellen, und
(5) Gewinnen des Antikörpers.

2. Verfahren nach Anspruch 1, wobei das Immunogen ein T-Zellstamm ist, welcher CD4-Antigen exprimiert, oder gereinigte T-Lymphozyten.

3. Verfahren nach Anspruch 1, wobei die Immunocytomzellen von Ratten oder Mäusen stammen.

4. Hybridzelle, die einen monoklonalen Antikörper der Ratte produziert, der an ein CD4-Humanantigenepitop bindet, welches ausreichend von demjenigen Epitop abgesetzt ist, das von monoklonalen Antikörpern der Maus gebunden wird, sodaß es nicht zur Kreuzblockierung kommt.

5. Hybridzelle nach Anspruch 4, wobei der monoklonale Antikörper der Ratte an ein CD4-Humanantigenepitop bindet, das von den Epitopen, die von den monoklonalen Antikörpern OKT4 und Leu3a der Maus definiert werden, verschieden ist.

6. Hybridzelle, die Antikörper produziert, welche die Bindungsspezifität von Antikörpern besitzen, die von der Hybridzelle LO-CD4-a mit der ECACC-Hinterlegungsnr. 89012101 produziert werden.

7. Hybridzelle, die Antikörper produziert, welche die Bindungsspezifität von Antikörpern besitzen, die von der Hybridzelle LO-CD4-b mit der ECACC-Hinterlegungsnr. 89012102 produziert werden.

8. Monoklonaler Antikörper der Ratte, der an ein CD4-Humanantigenepitop bindet, welches ausreichend von demjenigen Epitop abgesetzt ist, an das von Mäusen abgeleitete monoklonale Antikörper binden, daß es nicht zur Kreuzblockierung kommt.

9. Monoklonaler Antikörper der Ratte nach Anspruch 8, wobei der monoklonale Antikörper an ein CD4-Humanantigenepitop bindet, das von den Epitopen, die von den monoklonalen Antikörpern OKT4 und Leu3a der Maus definiert werden, verschieden ist.

10. Monoklonaler Antikörper der Ratte, der die Bindungsspezifität des monoklonalen Antikörpers LO-CD4-a besitzt, der von der Hybridzelle LO-CD4-a mit der ECACC-Hinterlegungsnr. 89012101 produziert wird.

11. Monoklonaler Antikörper der Ratte, der die Bindungsspezifität des monoklonalen Antikörpers LO-CD4-b besitzt, der von der Hybridzelle LO-CD4-b der mit ECACC-Hinterlegungsnr. 89012102 produziert wird.

12. Bindungsprotein, welches die Bindungsspezifität des monoklonalen Antikörpers LO-CD4-a besitzt, der von der Hybridzelle LO-CD4-a mit der ECACC-Hinterlegungsnr. 89012101 produziert wird.

13. Bindungsprotein, welches die Bindungsspezifität des monoklonalen Antikörpers LO-CD4-b besitzt, der von der Hybridzelle LO-CD4-b der mit ECACC-Hinterlegungsnr. 89012102 produziert wird.

14. Verwendung eines monoklonalen Antikörpers der Ratte nach einem der Ansprüche 8 bis 13 zur Herstellung eines Präparats für ein in vivo therapeutisches Verfahren.

15. In vitro diagnostisches Verfahren, das das In-Kontakt-Bringen eines monoklonalen Antikörpers der Ratte, der gemäß einem der Ansprüche 1-13 hergestellt worden ist, mit einer geeigneten Testprobe und Durchführung eines Assays auf das CD4-Antigen umfaßt.

## Patentansprüche (Patentansprüche für folgende(n) Vertragsstaat(en): ES)

1. Verfahren zur Herstellung monoklonaler Antikörper der Ratte, die an CD4-Humanantigen binden, welches folgende Schritte umfaßt:
(1) Immunisieren einer Ratte oder von immunokompetenten Rattenzellen in vitro mit einem Immunogen, welches CD4-Antigen umfaßt,
(2) Verschmelzen der immunisierten Zellen der Ratte oder der immunisierten immunokompetenten Rattenzellen mit Immunocytomzellen,
(3) Selektieren der Hybridzellen, die Antikörper produzieren, welche an ein CD4-Humanantigenepitop binden, welches ausreichend von demjenigen Epitop abgesetzt ist, an das von Mäusen abgeleitete monoklonale Antikörper binden, daß es nicht zur Kreuzblockierung kommt,
(4) Züchten der selektierten Hybridzellen, und
(5) Gewinnen des Antikörpers.

2. Verfahren nach Anspruch 1, wobei das Immunogen ein T-Zellstamm ist, der CD4-Antigen exprimiert, oder gereingte T-Lymphozyten.

3. Verfahren nach Anspruch 1, wobei die Immunocytomzellen von Ratten oder Mäusen stammen.

4. Verfahren nach Anspruch 1, wobei der monoklonale Antikörper an ein CD4-Humanantigenepitop bindet, das von den Epitopen verschieden ist, die von den monoklonalen Antikörpern OKT4 und Leu3a der Maus definiert werden.

5. Verfahren nach Anspruch 1, wobei der monoklonale Antikörper die Bindungsspezifität des monoklonalen Antikörpers LO-CD4-a besitzt, der von der Hybridzelle LO-CD4-a mit der ECACC-Hinterlegungsnr. 89012101 produziert wird.

6. Verfahren nach Anspruch 1, wobei der monoklonale Antikörper die Bindungsspezifität des monoklonalen Antikörpers LO-CD4-b besitzt, der von der Hybridzelle LO-CD4-b der mit ECACC-Hinterlegungsnr. 89012102 produziert wird.

7. Verfahren zur Herstellung einer Hybridzelle, die einen monoklonalen Antikörper der Ratte, der an CD4-Humanantigen bindet, produziert, welches folgende Schritte umfaßt:
(1) Immunisieren einer Ratte oder von immunokompetenten Rattenzellen in vitro mit einem Immunogen, welches CD4-Antigen umfaßt,
(2) Verschmelzen der immunisierten Zellen der Ratte oder der immunisierten immunokompetenten Rattenzellen mit Immunocytomzellen,
(3) Selektieren der Hybridzellen, die Antikörper produzieren, welche an ein CD4-Humanantigenepitop binden, welches ausreichend von demjenigen Epitop abgesetzt ist, an das von Mäusen abgeleitete monoklonale Antikörper binden, daß es nicht zur Kreuzblockierung kommt.

8. Verfahren nach Anspruch 7, wobei das Immunogen ein T-Zellstamm ist, der CD4-Antigen exprimiert, oder gereingte T-Lymphozyten.

9. Verfahren nach Anspruch 7, wobei die Immunocytomzellen von Ratten oder Mäusen stammen.

10. Verfahren nach Anspruch 7, wobei der monoklonale Antikörper der Ratte an ein CD4-Humanantigenepitop bindet, das von den Epitopen verschieden ist, die von den monoklonalen Antikörpern OKT4 und Leu3a der Maus definiert werden.

11. Verfahren nach Anspruch 7, wobei der monoklonale Antikörper die Bindungsspezifität der Antikörper besitzt, die von der Hybridzelle LO-CD4-a mit der ECACC-Hinterlegungsnr. 89012101 produziert werden.

12. Verfahren nach Anspruch 7, wobei der monoklonale Antikörper die Bindungsspezifität der Antikörper besitzt, die von der Hybridzelle LO-CD4-b mit der ECACC-Hinterlegungsnr. 89012102 produziert werden.

13. In vitro diagnostisches Verfahren, das das In-Kontakt-Bringen eines monoklonalen Antikörpers der Ratte, der gemäß einem der Ansprüche 1-6 hergestellt worden ist, mit einer geeigneten Testprobe und Durchführung eines Assays auf das CD4-Antigen umfaßt.

## Revendications (Revendications pour l'(les) Etat(s) contractant(s) suivant(s): AT, BE, CH, LI, DE, FR, GB, IT, NL)

1. Méthode de production d'anticorps monoclonaux de rat qui se lient à un antigène CD4 humain, comprenant les étapes selon lesquelles:
(1) on immunise un rat ou des cellules immunocompétentes de rat *in vitro* avec un immunogène comprenant un antigène CD4;
(2) on fusionne des cellules immunisées dudit rat ou des cellules immunocompétentes de rat immunisées avec des cellules d'immunocytome;
(3) on sélectionne les cellules d'hybridomes qui produisent un anticorps qui se lie à un épitope d'antigène CD4 humain suffisamment distinct de ceux qui sont fixés par les anticorps monoclonaux murins pour qu'il n'y ait pas de blocage croisé;
(4) on cultive les cellules d'hybridomes sélectionnées; et
(5) on récupère ledit anticorps.

2. Méthode selon la revendication 1, dans laquelle ledit immunogène est une lignée de lymphocytes T qui exprime un antigène CD4 ou des lymphocytes T purifiés.

3. Méthode selon la revendication 1, dans laquelle lesdites cellules d'immunocytome sont des cellules d'immunocytome de rat ou de souris.

4. Hybridome produisant un anticorps monoclonal de rat qui se lie à un épitope d'antigène CD4 humain suffisamment distinct de ceux qui sont fixés par les anticorps monoclonaux murins pour qu'il n'y ait pas de blocage croisé.

5. Hybridome selon la revendication 4, où ledit anticorps monoclonal de rat se lie à un épitope d'antigène CD4 humain autre que les épitopes définis par les anticorps monoclonaux murins OKT4 et Leu3a.

6. Hybridome qui produit des anticorps ayant la spécificité de liaison d'anticorps produits par l'hybridome LO-CD4-a ayant le n° de dépôt ECACC 89012101.

7. Hybridome qui produit des anticorps ayant la spécificité de liaison d'anticorps produits par l'hybridome LO-CD4-b ayant le n° de dépôt ECACC 89012102.

8. Anticorps monoclonal de rat qui se lie à un épitope d'antigène CD4 humain suffisamment distinct de ceux qui sont fixés par les anticorps monoclonaux murins pour qu'il n'y ait pas de blocage croisé.

9. Anticorps monoclonal de rat selon la revendication 8, où ledit anticorps monoclonal se lie à un épitope d'antigène CD4 humain autre que les épitopes définis par les anticorps monoclonaux murins OKT4 et Leu3a.

10. Anticorps monoclonal de rat ayant la spécificité de liaison de l'anticorps monoclonal LO-CD4-a produit par l'hybridome LO-CD4-a ayant le n° de dépôt ECACC 89012101.

11. Anticorps monoclonal de rat ayant la spécificité de liaison de l'anticorps monoclonal LO-CD4-b produit par l'hybridome LO-CD4-b ayant le n° de dépôt ECACC 89012102.

12. Protéine liante ayant la spécificité de liaison de l'anticorps monoclonal LO-CD4-a produit par l'hybridome LO-CD4-a ayant le n° de dépôt ECACC 89012101.

13. Protéine liante ayant la spécificité de liaison de l'anticorps monoclonal LO-CD4-b produit par l'hybridome LO-CD4-b ayant le n° de dépôt ECACC 89012102.

14. Utilisation d'un anticorps monoclonal de rat selon l'une quelconque des revendications 8 à 13 pour la production d'une préparation pour une méthode thérapeutique *in vivo*.

15. Méthode de diagnostic *in vitro* comprenant la mise en contact d'un anticorps monoclonal de rat produit selon l'une quelconque des revendications 1 à 13 avec un échantillon à analyser convenable, et le dosage de l'antigène CD4.

## Revendications (Revendications pour l'(les) Etat(s) contractant(s) suivant(s): ES)

1. Méthode de production d'anticorps monoclonaux de rat qui se lient à un antigène CD4 humain, comprenant les étapes selon lesquelles:
(1) on immunise un rat ou des cellules immunocompétentes de rat *in vitro* avec un immunogène comprenant un antigène CD4;
(2) on fusionne des cellules immunisées dudit rat ou des cellules immunocompétentes de rat immunisées avec des cellules d'immunocytome;
(3) on sélectionne les cellules d'hybridomes qui produisent un anticorps qui se lie à un épitope d'antigène CD4 humain suffisamment distinct de ceux qui sont fixés par les anticorps monoclonaux murins pour qu'il n'y ait pas de blocage croisé;
(4) on cultive les cellules d'hybridomes sélectionnées; et
(5) on récupère ledit anticorps.

2. Méthode selon la revendication 1, dans laquelle ledit immunogène est une lignée de lymphocytes T qui exprime un antigène CD4 ou des lymphocytes T purifiés.

3. Méthode selon la revendication 1, dans laquelle lesdites cellules d'immunocytome sont des cellules d'immunocytome de rat ou de souris.

4. Méthode selon la revendication 1, dans laquelle ledit anticorps monoclonal de rat se lie à un épitope d'antigène CD4 humain autre que les épitopes définis par les anticorps monoclonaux murins OKT4 et Leu3a.

5. Méthode selon la revendication 1, dans laquelle ledit anticorps monoclonal de rat a la spécificité de liaison de l'anticorps monoclonal LO-CD4-a produit par l'hybridome LO-CD4-a ayant le n° de dépôt ECACC 89012101.

6. Méthode selon la revendication 1, dans laquelle ledit anticorps monoclonal de rat a la spécificité de liaison de l'anticorps monoclonal LO-CD4-b produit par l'hybridome LO-CD4-b ayant le n° de dépôt ECACC 89012102.

7. Méthode de production d'un hybridome qui produit un anticorps monoclonal de rat qui se lie à un antigène CD4 humain, comprenant les étapes selon lesquelles:
(1) on immunise un rat ou des cellules immunocompétentes de rat *in vitro* avec un immunogène comprenant un antigène CD4;
(2) on fusionne des cellules immunisées dudit rat ou des cellules immunocompétentes de rat immunisées avec des cellules d'immunocytome;
(3) on sélectionne les cellules d'hybridomes qui produisent un anticorps qui se lie à un épitope d'antigène CD4 humain suffisamment distinct de ceux qui sont fixés par les anticorps monoclonaux murins pour qu'il n'y ait pas de blocage croisé.

8. Méthode selon la revendication 7, dans laquelle ledit immunogène est une lignée de lymphocytes T qui exprime un antigène CD4 ou des lymphocytes T purifiés.

9. Méthode selon la revendication 7, dans laquelle lesdites cellules d'immunocytome sont des cellules d'immunocytome de rat ou de souris.

10. Méthode selon la revendication 7, dans laquelle ledit anticorps monoclonal de rat se lie à un épitope d'antigène CD4 humain autre que les épitopes définis par les anticorps monoclonaux murins OKT4 et Leu3a.

11. Méthode selon la revendication 7, dans laquelle ledit anticorps monoclonal de rat a la spécificité de liaison d'anticorps produits par l'hybridome LO-CD4-a ayant le n° de dépôt ECACC 89012101.

12. Méthode selon la revendication 7, dans laquelle ledit anticorps monoclonal de rat a la spécificité de liaison d'anticorps produits par l'hybridome LO-CD4-b ayant le n° de dépôt ECACC 89012102.

13. Méthode de diagnostic *in vitro* comprenant la mise en contact d'un anticorps monoclonal de rat produit selon l'une quelconque des revendications 1 à 6 avec un échantillon à analyser convenable, et le dosage de l'antigène CD4.
